Europäisches Patentamt

European Patent Office

Office européen des brevets

(19)

(11) **EP 1 516 900 A1**

## (12) EUROPEAN PATENT APPLICATION
published in accordance with Art. 158(3) EPC

(43) Date of publication:
**23.03.2005 Bulletin 2005/12**

(21) Application number: **03742860.4**

(22) Date of filing: **04.04.2003**

(51) Int Cl.[7]: **C09K 5/16**, A61F 7/08

(86) International application number:
**PCT/JP2003/004363**

(87) International publication number:
**WO 2003/097764 (27.11.2003 Gazette 2003/48)**

(84) Designated Contracting States:
**AT BE BG CH CY CZ DE DK EE ES FI FR GB GR HU IE IT LI LU MC NL PT RO SE SI SK TR**

(30) Priority: **20.05.2002 JP 2002145517**

(71) Applicant: **Mycoal Warmers Co., Ltd**
**Tochigi-Shi Tochigi 328-0067 (JP)**

(72) Inventors:
• **USUI, Kaoru Mycoal Warmers Co., Ltd.**
**Tochigi-shi, Tochigi 328-0067 (JP)**

• **AIDA, Michio Mycoal Warmers Co., Ltd.**
**Tochigi-shi, Tochigi 328-0067 (JP)**
• **KIMURA, Hisao Mycoal Warmers Co., Ltd.**
**Tochigi-shi, Tochigi 328-0067 (JP)**
• **DODO, Toshihiro Mycoal Warmers Co., Ltd.**
**Tochigi-shi, Tochigi 328-0067 (JP)**

(74) Representative: **Körfer, Thomas, Dipl.-Phys.**
**Mitscherlich & Partner,**
**Patent- und Rechtsanwälte,**
**Sonnenstrasse 33**
**80331 München (DE)**

## (54) HEATING COMPOSITION AND HEATING ELEMENT

(57) There are provided a heat-generating composition excellent in moldability, shape retaining property and heat-generating characteristics, and a heat-generating body using the same that can maintain the shape of a molded article of the heat-generating composition irrespective to the pressure inside the container bag and has excellent heat-generating characteristics and long-term heat generation maintaining property. It is a non-viscous heat-generating composition having excessive water **characterized in that** the heat generating composition generating heat in contact with air comprises, as essential components, a water absorbing polymer, a carbon component, a heat-generating promoter, water and a heat-generating substance, has excessive water, and has an incremental degree of viscosity of less than 1,000 cP and a liquid permeation degree of 5 or more.

Fig. 1

EP 1 516 900 A1

## Description

(Technical Field)

The present invention relates to a heat-generating composition and a heat-generating body, and more particularly, it relates to a heat-generating composition that contains, as essential components, a water absorbing polymer, activated carbon, a heat-generation promoter, water, a water retaining agent and a heat-generating substance, and that has excellent moldability, shape retaining property and heat-generating characteristics, and a heat-generating body that has excellent shape retaining property and long-term heat-generation persistence.

(Background Art)

In order to extend the heat-generating time, it is necessary that the heat-generating composition has appropriate water holding property, air permeability and the like thereinside. Accordingly, various heat-generating compositions using a water absorbing polymer have recently been proposed. In the case where powder of a high water absorbing resin is used as a water retaining agent, however, because the particle diameter thereof is generally as large as close to 1 mm, the powder is difficult to be formed, the water retaining agent is adhered to each other through gelation with water, and the viscosity of the heat-generating composition is increased, whereby problems are liable to occur in heat-generating performance.

As a measure for improving the handleability, various heat-generating compositions have been proposed in that a water absorbing polymer is formed into a composite material with a heat-generating substance, activated carbon, water and a thickener to attain shape retaining property and maintenance of heat-generating characteristics. For example, JP-A-H4-293989 proposes a process for producing a heat-generating composition of a metallic hydroxide heat-generating composition containing metallic powder, a halide, water and a water absorption assistant, in which from 55 to 70 parts by weight of water is mixed with 100 parts by weight of the metallic powder, and a water absorption agent can retain added water, by mixing the water absorbing agent, and then granulated to an average particle diameter of 0.5 mm or more, and a process for producing a heat-generating composition that is improved in particle strength after granulation by mixing from 10 to 20 parts by weight of a adhesive binder with the added water, whereby the flowability of the raw material of the heat-generating composition containing the metallic powder, the halide, the water and the water absorbing agent is improved, and troubles upon cutting out and charging from a hopper are prevented, the productivity is improved, and the heat-generating duration is extended without causing increase in volume.

JP-A-H6-343658 proposes a disposal body warmer containing a heat-generating composition generating heat in the presence of air containing metallic powder, such as iron powder and the like, a reaction assistant, such as a metallic halide, a metallic sulfate salt and the like, water, a water retaining agent, such as activated carbon, vermiculite, silica gel, wood powder, a water absorbing polymer and the like, and a powder thickener, such as corn starch, potato starch and the like, added to each other, which is placed in a bag having air permeability formed with an air nonpermeable packing material having air permeable holes or an air permeable part provided, having air permeability on one surface of the bag having air permeability and a cohesive agent layer on the other surface, whereby such a disposable body warmer is provided that is prevented from deviation of the heat-generating composition to one side in the bag of the body warmer, is good in fitting feeling when it is worn by being attached to the body or onto an underwear, satisfies temperature characteristics that are demanded as a disposable body warmer and causes less variation in heat generation among the individual products, thus is stable in the quality.

JP-A-S59-189183 proposes a solid heat-generating composition containing a powder heat-generating composition mixed with a binder, such as CMC and the like, followed by subjecting to compression molding. WO2000-13626 proposes a flexible heat-generating body using no binder but using a water absorbing polymer, in which at least a part of the water absorbing polymer is broken under high pressure to make it attain the same role as a binder to bind the composition. However, the range of attaining practical heat-generating characteristics is narrow, and only a very small heat-generating body can be obtained, but a heat-generating body having a size of a commercially available product or larger is considerably deteriorated in heat-generating characteristics and is difficult to attain long-term heat generation, so as to provide poor practicability.

JP-A-H9-75388 proposes a heat-generating composition formed into an ink or cream form in total containing, as essential components, a heat-generating substance, a water absorbing polymer and/or a thickener, a carbon component and/or a metallic chloride, and water, a heat-generating body using the heat-generating composition in an ink or cream form, and a process for producing them, whereby such a process for producing a heat-generating body is provided that prevents formation of powder dusts upon producing the heat-generating body, suppresses a heat-generating reaction of the heat-generating composition to prevent loss due to the heat-generating reaction and deterioration in quality and solidification of the heat-generating composition upon production, and is prevented from migration and deviation of the heat-generating composition by transferring the heat-generating composition to a water absorbing film or sheet, a nonwoven fabric, a fabric or a porous foamed film or sheet, or in alternative, to a water absorbing layer provided thereon to distribute uniformly and fix the heat-generating composion in and to a bag member.

In the case where a high water absorbing resin is applied to a heat-generating composition having excessive water to realize a heat-generating composition that is excellent in moldability and attain long-term heat generation, the high water absorbing polymer swells and increases in volume due to absorption of a salt water and deterioration in liquid permeability due to increase in viscosity, and as a result, long-term heat generation of a heat-generating composition having excessive water cannot be realized. That is, the water holding capability of the conventional water retaining agent, such as wood powder, vermiculite and the like, is short with respect to the volume thereof, and thus, the use of the water absorbing polymer is being mainstream of the water retaining agent for a heat-generating composition. This sufficiently satisfies the effect as a water retaining agent. However, in the case where water is present in such an amount that is near the water holding capacity or more than the capacity, it not only is expanded to impair the moldability, but also is dissolved to become pasty in total to cause such a problem in that the liquid containing the polymer becomes viscous to impair the heat-generating performance of the heat-generating composition in a serious extent.

Therefore, it is the current situation that what are available are only a powder or semi-kneaded heat-generating composition formed by restricting the added water to the necessary limit, such as a powder material, semi-kneaded material, an ink-form material, a cream-form material, a paste material or the like, that is excellent in heat-generating performance but has problems in moldability, or a viscous heat-generating composition formed by adding a thickener that is excellent in moldability but has problems in heat-generating performance.

A water absorbing polymer having a large water absorbing amount is necessary for prolonging the heat-generating duration per the same volume because the volume occupied by the heat-generating composition in the heat-generating body formed by placing the heat-generating composition in a container bag is almost decided. Therefore, a high water absorbing resin necessarily has a large absorption capacity, i.e., the amount of an aqueous solution that can be absorbed by the unit volume of the resin is necessarily large, and other various characteristics are required therefor.

Good liquid permeability in the swollen state can be exemplified as one of them. That is, a water absorbing polymer is generally softened through swelling upon absorbing an aqueous liquid, and in the case where the resin particles cannot retain the shape thereof due to significant softening to be close to a fluid state, the liquid permeability is deteriorated to impair subsequent drainage. This is because the components of the heat-generating composition in the heat-generating body are distributed in a lamellar form to have a constant thickness in the drainage direction of the liquid, and in the case where the water absorbing polymer particles in the vicinity of the surface are softened through swelling by absorbing water to clog the gaps among the components and the resin particles, migration of excessive water inside and outside is restricted, and the aqueous solution is prevented from penetrating in the water retaining agent and from discharging to the exterior, whereby the migration velocity of the excessive water is considerably lowered.

In order to improve the liquid permeability, the crosslinking degree of the water absorbing polymer particles is increased so that the resin particles are hard and are difficult to be deformed, but the high crosslinking degree prevents the resin particles from swelling to reduce the absorption capacity.

The invention is to solve the problems of a heat-generating composition having excessive water and containing a water absorbing polymer, and an object thereof is to provide such a heat-generating composition that uses a water absorbing polymer, is excellent in moldability and shape holding property enabling high-speed formation of various shapes, is excellent in heat-generating temperature, and can produce a heat-generating body having a long heat-generating duration.

(Disclosure of the Invention)

As a result of earnest investigations made by the inventors for solving the problems, it has been found that a non-viscous heat-generating composition containing, as an essential component, a water absorbing polymer having a large water absorbing and water retaining amount, having a high liquid permeability and having excessive water can retain the moldability, the shape retaining property and the heat-generating characteristics to high levels and can provide a long-term heating effect.

That is, the non-viscous heat-generating composition having excessive water of the invention, as described in claim 1, is a heat-generating composition having excessive water characterized in that the heat-generating composition generating heat in contact with air comprises, as essential components, a water absorbing polymer, a carbon component, a heat-generating promoter, water and a heat-generating substance, has excessive water, and has an incremental degree of viscosity of 1,000 cP or less and a liquid permeability of 15 or more.

The non-viscous heat-generating composition having excessive water described in claim 2 is a non-viscous heat-generating composition having excessive water as described in claim 1, characterized in that the non-viscous heat-generating composition having excessive water contains a water retaining agent.

The non-viscous heat-generating composition having excessive water described in claim 3 is a non-viscous heat-generating composition having excessive water as described in claim 1, characterized in that the water retaining agent is at least one kind selected from wood powder, terraballoon and Shirasu balloon.

The non-viscous heat-generating composition having excessive water described in claim 4 is a non-viscous heat-generating composition having excessive water as described in claim 1, characterized in that the non-viscous heat-generating composition having excessive water contains at least one kind selected from a surfactant, a defoaming agent, a pH adjusting agent, a hydrophobic polymer compound, a pyroelectric substance, a far infrared ray radiating substance, a negative ion-generating substance, a hydrogen generation suppressing substance, an organic silicon compound, a water soluble polymer, a thickener, a binder, an excipient, an aggregating agent, a soluble adhesive material, an aggregate, a fibrous material, a medical or sanitary agent, a fertilizer component and a heat-generating assistant.

The non-viscous heat-generating composition having excessive water described in claim 5 is a non-viscous heat-generating composition having excessive water as described in claim 1, characterized in that water insoluble solid components other than a high water absorbing resin in the non-viscous heat-generating composition having excessive water has an average particle diameter of 150 µm or less.

The non-viscous heat-generating composition having excessive water described in claim 6 is a non-viscous heat-generating composition having excessive water as described in claim 1, characterized in that the non-viscous heat-generating composition having excessive water has a shape retaining degree of 70 or more.

The heat-generating body of the invention, as described in claim 7, is characterized by placing a non-viscous heat-generating composition having excessive water as described in claim 1 in a container bag having air permeability in at least a part thereof.

The heat-generating body described in claim 8 is a heat-generating body as described in claim 7, characterized in that a molded article of the heat-generating composition in the heat-generating body has a shape retaining degree of 70 or more.

The heat-generating body described in claim 9 is a heat-generating body as described in claim 7, characterized in that at least a part of the container bag has water absorbing property.

The heat-generating body described in claim 10 is a heat-generating body as described in claim 7, characterized in that at least one kind selected from iron powder, a carbon component, a fibrous material, a binder, a thickener, an excipient, an aggregating agent, a soluble cohesive material, a far infrared ray radiating substance, a negative ion-generating substance, a pyroelectric substance, an organic silicon compound, a water absorbing agent, a high water absorbing polymer, a water-separation preventing stabilizer and a medical or sanitary agent is laminated, diffused or coated on one surface or both surfaces of a molded article of the heat-generating composition.

The heat-generating body described in claim 11 is a heat-generating body as described in claim 7, characterized in that at least one kind selected from a far infrared ray radiating substance, a negative ion-generating material, a pyroelectric substance and a medical or sanitary agent is laminated with, coated on or contained in at least one kind or at least a part of a base material and a covering material constituting the container bag.

The heat-generating body described in claim 12 is a heat-generating body as described in claim 7, characterized in that unevenness is formed on the whole surface or a part surface of a molded article of the heat-generating composition.

The heat-generating body described in claim 13 is a heat-generating body as described in claim 7, characterized in that unevenness is formed on at least the whole surface or a part surface of a molded article of the heat-generating composition and a material laminated on the heat-generating composition.

The heat-generating body described in claim 14 is a heat-generating body as described in claim 7, characterized in that a adhesive agent layer or a gel layer is laminated on at least a part of an exposed surface of one of the base material and the covering material.

The heat-generating body described in claim 15 is a heat-generating body as described in claim 14, characterized in that the adhesive agent layer or the gel layer is a wet compress layer containing a wet compress agent, or a drug-containing layer laminated with, coated with, containing or carrying at least one kind of a far infrared ray radiating substance, a negative ion-generating substance, a pyroelectric substance and a medical or sanitary agent.

The non-viscous heat-generating composition having excessive water of the invention maintains flowability, moldability, shape retaining property and heat-generating characteristics to high levels by using water as a binder to bind the respective components through the surface tension of water present among the components, and is quite different from the viscous heat-generating composition pursuing only moldability and shape retaining property under sacrifice of heat-generating characteristics by binding the respective components with the conventional adhesive, such as a thickener agent or the like. The non-viscous heat-generating composition having excessive water of the invention is such a heat-generating composition that can retain moldability and shape retaining property with maintenance of heat-generating characteristics and can provide heat-generating bodies excellent in heat-generating characteristics having various kinds of shapes.

Because the non-viscous heat-generating composition having excessive water of the invention has high liquid permeability and contains a water absorbing polymer as an essential component, a necessary amount of water for heat generation can be assured in a good heat-generating environment, and thus a long-term heat generation at a desired temperature can be realized.

Because the non-viscous heat-generating composition having excessive water of the invention generates heat by adjusting the amount of excessive water to an appropriate value, it can be formed into a heat-generating body by containing in a container bag under such conditions that it is substantially dehydrated to a level where heat generation is carried out in the air, in such a manner that a water absorbing container bag is used, and the excessive water is absorbed by the container bag, or in alternative, in the case where the container bag is water non-absorbing, such measures are carried out as a physical forced drainage by compression, decompression or compression and decompression of the heat-generating composition or a molded article thereof, water radiation by allowing to stand in a space, and the like measures.

In the non-viscous heat-generating composition having excessive water of the invention, the term "having excessive water" means that the water mobility value is from 3 to 50, and the term "non-viscous" means that the incremental degree of viscosity is less than 1,000 cP.

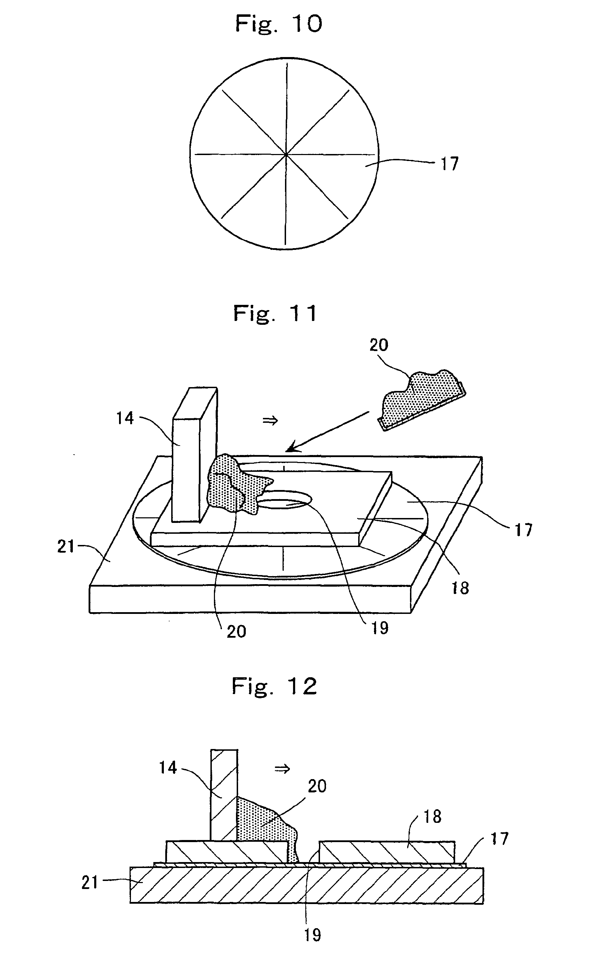

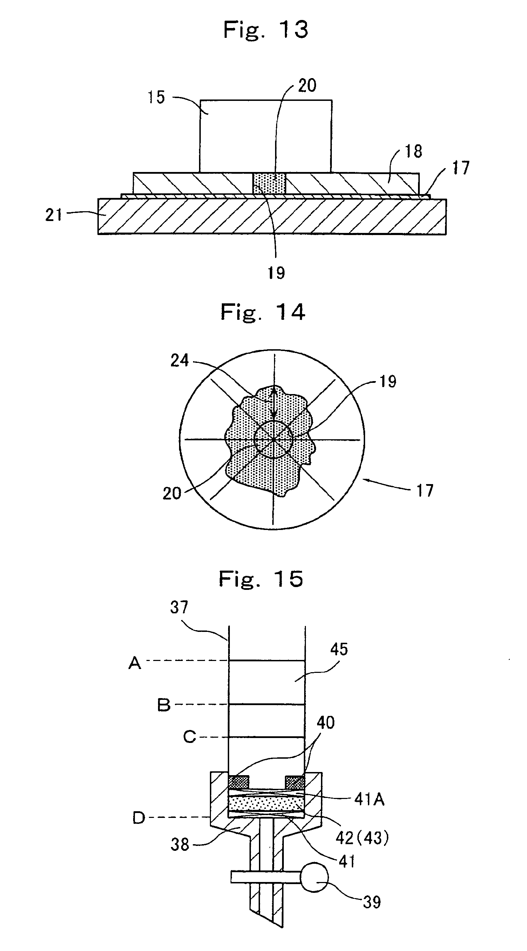

The water mobility value is a value showing an amount of excessive water that can be moved outside the composition among the water content of the heat-generating composition. The water mobility value will be described with reference to Figs. 10 to 14. As shown in Fig. 10, No. 2 filter paper 17, on which eight lines extending from the center with intervals of 45° have been written, is placed on a stainless steel plate 21 as shown in Figs. 11 and 12, and a template 18 having a hole 19 of a hollow cylinder shape having an inner diameter 200 mm and a height of 4 mm is placed on the center of the filter paper 17. A sample 20 is placed in the vicinity of the hole 19 having a hollow cylinder shape, and a pressing plate 14 is moved along the template 18, so as to place the sample 20 into the hole 19 having a hollow cylinder shape with pressing (press molding). Furthermore, as shown in Fig. 13, the hole 19 of a hollow cylinder shape having the sample 20 therein and the periphery thereof are covered with a wind guard 15, and they are maintained for 5 minutes. Thereafter, the filter paper 17 is brought out (Fig. 14), and the excursion of soaking of water or an aqueous solution is read as distances 24 from the circumference part, which is an edge of the hole 19, to the front edge of soaking along the radial line in terms of mm units. The distances 24 along the respective lines are read to obtain eight values in total. The thus-read eight values are designated as measured water content values (a, b, c, d, e, f, g and h).

An arithmetic average of the eight measured water content values is designated as a water content value (mm) of the sample.

A water content for measuring the true water content value is a blended water amount of the heat-generating composition corresponding to the weight of the heat-generating composition having a diameter of 20 mm and a height of 4 mm, and the measurement is carried out by using only water corresponding to the water amount to obtain a true water content value (mm) through the similar calculation. A value obtained by dividing the water content value by the true water content value is multiplied with 100 to obtain the water mobility value.

That is, water mobility value = (water content value (mm)/true water content value (mm)) x 100.

The water mobility value herein is a value upon lamination, for example, by pressing molding or the like.

The water mobility value (0 to 100) of the heat-generating composition of the invention is generally from 3 to 50, preferably from 6 to 35, and more preferably from 6 to 20. In the case where it is less than 3, when the composition is laminated on a base material through a mold, it cannot be laminated due to poor flowability, and in the case where it exceeds 50, the composition runs off the mold shape to fail to maintain the shape.

The incremental degree of viscosity shows a difference between a BH type viscosity (BH type) S of the heat-generating composition containing a heat-generating substance, a carbon component, an oxidation accelerator and water, and a BH type viscosity (BH type) T of a heat-generating composition obtained by adding other substances thereto, and the value T-S is generally less than 1, 000 cP (centipoise) , preferably less than 500 cP, and more preferably less than 300 cP, which includes 0 and a negative value. There is no limitation in the negative value, and thus the viscosity may be decreased by any extent. As using a BH type viscometer (BH type) for measuring the BH type viscosity, such a value is employed that is obtained by placing a No. #7 rotor at 2 rpm in a center of a sample to obtain a value in a stable state after lapsing 5 minutes or more from the start of rotation. A BH type viscometer (BH type) with a No.#7 rotor at 2 rpm has a full scale of 200,000 cP.

In the case where the value T-S is 1,000 cP or more, adverse influences occur in heat-generating characteristics, such as considerable deterioration in heat-generating property.

The absorption capacity is a value obtained in the following manner. 0. 50 g (W1 g) of a resin is weighed and placed in a nylon bag of 250 mesh (20 cm x 10 cm), and it is immersed in 500 mL of a reaction promoter aqueous solution (concentration: 11% by weight) at room temperature for 30 minutes. Subsequently, the nylon bag is taken out, followed by draining by hanging for 15 minutes, and the resin in the bag is placed on a non-water absorbale polyethylene film. After absorbing aqueous solution around the resin with filter paper, the resin is collected with tweezers, and the weight (W2 g) of the resin absorbing water is measured to obtain the value by the following equation. Absorption capacity of reaction accelerator = W2/W1 (g/g)

The average particle diameter is a value obtained in the following manner. Firstly, sieves and trays of the ASTM standard of, for example, 8, 12, 20, 32, 42, 60, 80, 100, 115, 150, 200, 250 and 280 mesh are arranged in this order from above. About 50 g of resin particles are placed on the uppermost 8 mesh sieve, and it is shaken with a rotap automatic shaker for 1 minute. The weights of the resin particles on the respective sieves and trays are measured, and the particle diameter distribution is obtained in terms of weight fraction with the total weight being 100%. The particle diameter at 50% in terms of weight fraction is designated as an average particle diameter. The sieves of the foregoing meshes may be used in combination of sieves of other meshes.

The liquid permeability is an index of the heat-generating environment, and the heat-generating environment is better when the value is larger. That is, it shows an extent of transmission and migration of water or an aqueous solution in a sample, and in the case where the value is large, the excessive water in the heat-generating composition is easily moved and discharged, and the traces formed by discharging the excessive water become air permeation paths, so as to mean that the heat-generating composition or a molded article thereof has good heat-generating environment.

As shown in Fig. 15, a cylindrical glass column 37 (column inner diameter: 50 mm, length: 500 mm) having a valve 39 at a lower end is prepared.

A measuring sample having a molded article of a heat-generating composition 42 or 43 having a thickness of WT mm and an inner diameter of from 20 to 45 mm sandwiched with filters 41 and 41A having an outer diameter of 45 mm formed from a pulp nonwoven fabric is placed on a column pedestal 38, and a torus-shape filter retainer 40 having an outer diameter of 45 mm, an inner diameter of 20 mm and a thickness of 5 mm formed with polyvinyl chloride is placed thereon, followed by setting the column 37 thereon. Subsequently, 200 mL (A) of a saline solution 45 as a sample liquid is placed in the column 37. After allowing to stand for 5 minutes, the valve 39 is opened to measure the period of time (second) for passing the liquid level of the saline solution from the 150 mL (B) line to the 100 mL (C) line, and the liquid permeation rate (mL/sec) is calculated.

Furthermore, the liquid permeability R is calculated by the following equation.

$$WS \ (or \ WO) = 50 \ mL/passing \ time \ (sec)$$

$$WW = WS \times WT$$

$$R = (WW/WO) \times 100$$

WO:　liquid permeation rate in the absence of sample
WS:　liquid permeation rate in the presence of sample
WT:　thickness of sample (mm)
WW:　liquid permeation rate per 1 mm in thickness

The pulp nonwoven fabric is not limited as far as it has a large liquid permeability and can capture fine particles, and examples thereof include J-SOFT JS45HB-W, a trade name, produced by Havix Co., Ltd. and J-SOFT Co., Ltd. (basis weight: 45 $g/m^2$, paper thickness: 0.88 mm, water absorption magnification: 25 to 30).

Saline solution: 11% sodium chloride aqueous solution

The liquid permeability R herein is preferably 5 or more, more preferably 8 or more, and further preferably 10 or more. When in is less than 5, the excessive water remains in an inappropriate amount due to deteriorated draining property to deteriorate the heat-generating characteristics.

As a method for producing a sample from the heat-generating composition, a mold having a thickness of 1 mm and an inner diameter of 20 mm is placed on a filter having an outer diameter of 45 mm formed with a pulp nonwoven fabric, and the heat-generating composition is molded by leveling to form a molded article of the heat-generating composition having a thickness of 1 mm and an inner diameter of 20 mm on the filter. A filter having an outer diameter of 45 mm formed with a pulp nonwoven fabric is further placed on the molded article to obtain a sample for measurement.

The shape retaining degree is calculated by examining one independent heat-generating body having a heat-generating composition sealed around the full circumference as an object. In the case where there are plural bodies, an arithmetic average of shape retaining degrees of the respective independent heat-generating bodies is designated.

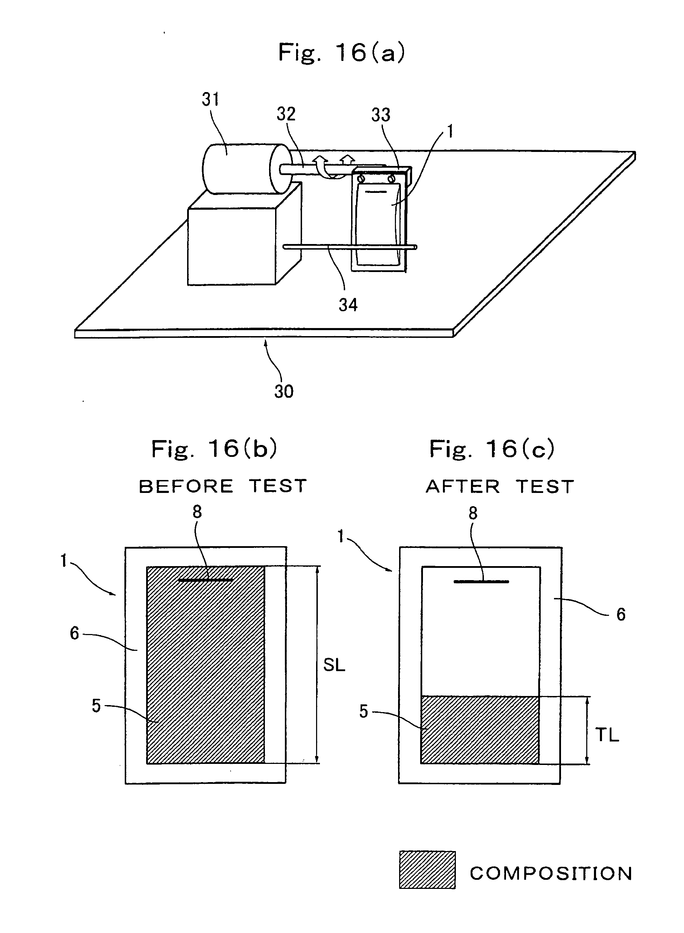

Explanation will be made with reference to Fig. 16. A heat-generating body 1 to be measured is placed on a level place, and after confirming that a heat-generating composition is substantially uniformly present in a heat-generating part, the maximum length SL of the heat-generating part is measured. In the case where there is nonuniformity, it is uniformized.

As shown in Fig. 16(a), the heat-generating body 1 is fixed on a fixed plate 33 fixed on a rotation axis 32 that is rotatable with a driving source 31 of a testing machine 30. The fixed position is an upper tip end of a covering material 6 of the heat-generating body 1 where no heat-generating composition is present. A slit 8 having a length of 10 mm is made on an air permeable surface of the covering material 6 at a position of 5 mm lower from the upper end of the heat-generating part, so that the heat-generating composition 2 is under the same pressure as the outer atmospheric pressure (see Fig. 16(b)). Thereafter, the fixed plate 33 is reciprocated by one reciprocation per second at a movement angle of 60° with rotation of the rotation axis 32, whereby the heat-generating body 1 is subjected to pendulum motion according thereto. At this time, it is made that at least a part of the heat-generating part runs on a sample beating member 34. After 10 reciprocations, the maximum length TL in vertical direction of the heat-generating composition is measured in the region of the heat-generating part occupied by the heat-generating composition with the body being fixed on the fixing plate 33 (fig. 16(c)).

The shape retaining degree (K) herein is defined as follows.

In the case where the heat-generating body is constituted from a single independent heat-generating body:

$$K = 100 \times TL/SL \qquad (1)$$

K: shape retaining degree
SL: maximum length of heat-generating composition on heat-generating part in horizontal direction before forming slit
TL: maximum length of heat-generating composition on heat-generating part in vertical direction after testing

In the case where the heat-generating body is constituted from plural independent heat-generating bodies:

$$Km = (K1 + K2 + ... + Kn)/n$$

Kn: shape retaining degrees of respective independent heat-generating bodies obtained by equation (1)

The shape retaining degree K is generally 70 or more, preferably 80 or more, and more preferably 90 or more.

In the case where the heat-generating body is constituted from plural independent heat-generating bodies, heat-generating compositions contained in all the independent heat-generating bodies constituting the heat-generating body are measured, and the number average value of the shape retaining degrees of the respective heat-generating bodies is generally 70 or more, preferably 80 or more, and more preferably 90 or more.

(Best Mode for carrying out the Invention)

The invention is applied to a heat-generating body that generates heat upon contact with oxygen in air. The invention is mainly used for heat-retention of a human body, and is also applied to a heat-generating body for heat-retention of pets and machines.

The heat-generating composition having excessive water of the invention has a water absorbing polymer, a heat-generating substance, a carbon component, a reaction promoter and water as essential components, and has excessive water, and it is a non-viscous heat-generating composition that initiates a heat-generating reaction by absorbing and/or removing a certain amount of excessive water.

Furthermore, the non-viscous heat-generating composition having excessive water may contain at least one kind selected from a surfactant, a defoaming agent, a pH adjusting agent, a hydrophobic polymer compound, a pyroelectric substance, a far infrared ray radiating substance, a negative ion-generating substance, a hydrogen generation suppressing substance, an organic silicon compound, a water soluble polymer, a thickener, a binder, an excipient, an aggregating agent, a soluble cohesive material, an aggregate, a fibrous material, a medical or sanitary agent, a fertilizer component and a heat-generating assistant.

In the non-viscous heat-generating composition having excessive water of the invention, the mixing proportions are not particularly limited, and it is preferred that 100 parts by weight of the heat-generating substance is mixed with from 0.01 to 20 parts by weight of the water absorbing polymer, from 1.0 to 50 parts by weight of the carbon component, from 1.0 to 50 parts by weight of the reaction promoter, from 0.01 to 10 parts by weight of a water retaining agent, from 0.01 to 5 parts by weight of the hydrogen generation suppressing agent, from 0.01 to 5 parts by weight of the surfactant, from 0.01 to 5 parts by weight of the defoaming agent, from 0.01 to 5 parts by weight of the pH adjusting agent, from 0.01 to 5 parts each by weight of the hydrophobic polymer compound, the aggregate, the fibrous material, the pyroelectric substance, the far infrared ray radiating substance, the negative ion-generating substance and the organic silicon compound, from 0.01 to 3 parts by weight each of the water soluble polymer, the thickener, the binder, the excipient, the aggregating agent, and the soluble adhesive material, from 0.01 to 1 parts by weight each of the medical or sanitary agent, the fertilizer component and the heat-generating assistant.

Any heat-generating substance can be used that generates heat upon contact with air, and a metal is generally used. For example, iron powder, zinc powder, aluminum powder, magnesium powder, alloy powder containing at least one of the metals, mixed metallic powder containing at least one of the metals, and the like are used, and among the metallic powder, iron powder is preferably used since it is the most excellent in overall properties, such as safety, handling property, cost, storage property, stability and the like. Examples of the iron powder include cast iron powder, atomized iron powder, electrolytic iron powder, reduced iron powder and the like. Furthermore, the iron powder containing carbon is also useful.

In particular, iron powder coated on a part of the surface of the iron powder with from 0.3 to 3.0% by weight of an electroconductive carbon substance is useful. Examples of the electroconductive carbon substance include carbon black, activated carbon and the like, with examples of the iron powder including reduced iron powder, atomized iron powder and sponge iron powder, and in particular, the case where the electroconductive carbon substance is activated carbon, and the iron powder is reduced iron powder is useful for a chemical body warmer.

As a method for coating the carbon component on the iron powder in this case, cathode thin film formation can be carried out by a coating treatment in a ball mill, a conical blender or the like for from 30 minutes to 3 hours. Specific examples thereof include such a method that from 0.1 to 10 parts by weight of the carbon component is used per 100 parts by weight of the iron powder, which are kneaded in an extruding mixer (AM-15F, produced by Hosokawamicron Corp., or the like) at a rotation number of from 500 to 1,500 rpm for from 10 to 80 minutes.

Examples of the carbon component include carbon black, graphite, activated carbon and the like. Activated carbon prepared from husks of coconuts, wood, charcoal, coal, bone charcoal or the like is useful, and those prepared from other rawmaterials, such as an animal originated substance, a natural gas, a fat, an oil and a resin, are also useful in the heat-generating composition of the invention. The species of the activated carbon is not limited, and activated carbon exerting excellent absorption maintenance property is preferred. The performance of the carbon component is preferably an iodine absorbing capability of from 550 to 1,200 mg/g and a methylene blue decoloring capability of from 60 to 300 mg/g, and more preferably an iodine absorbing capability of from 800 to 1,200 mg/g and a methylene blue decoloring capability of from 100 to 300 mg/g. A mixture of carbon may be used in the invention.

The oxidation promoter may be any material that can promote oxidation of the heat-generating substance. Examples thereof include a metallic halogenide, such as sodium chloride, potassium chloride, magnesium chloride, calcium chloride, ferrous chloride, ferric chloride, cupric chloride, manganese chloride, cuprous chloride and the like, a metallic sulfate, such as potassium sulfate, sodium sulfate, magnesium sulfate, calcium sulfate, copper sulfate, ferrous sulfate, ferric sulfate andmanganese sulfate, a nitrate, such as sodiumnitrate, potassium nitrate and the like, an acetate, such as sodium acetate and the like, and a carbonate, such as ferrous carbonate and the like. These may be used solely or combination.

The oxidation promoter is generally used in the form of an aqueous solution and may also be used in the form of powder as it is.

The water may be that from a suitable source. The purity and the kind thereof are not limited.

The water absorbing polymer is not particularly limited as far as it has water holding property and water absorbing property with respect to water or an aqueous solution and is insoluble in water or an aqueous solution at 50°C or lower, and it particularly preferably has such a absorption capacity that a water absorbing amount for a 11% saline aqueous solution of 5 g/g or more under consideration of swelling or the like through absorption of water.

In the case where the water absorbing amount for an 11% saline aqueous solution is less than 5 g/g, the heat-generating duration is shortened due to the small water absorbing amount.

The kind of the polymer and the polymerization method are not limited. Any material can be used irrespective to the production method of the water absorbing resin, such as polymerization of a component monomer in the presence of a crosslinking agent, partial crosslinking, surface crosslinking, crosslinking after gelation, and the like. The shape is also not particularly limited, and those having a spherical shape, a grape shape formed by combining plural spheres, an irregular shape, a fibrous shape and the like may be used.

In the case where the water absorbing polymer is a mixture or a composite material of resins having different water absorption rates, the ratio of the water absorption rates of the water absorbing resin having the largest water absorption rate and the water absorbing resin having the smallest water absorption rate is not particularly limited and is preferably 3 times or more.

Examples of the water absorbing polymer include a polyacrylate salt series, a polyvinyl alcohol series, a polyvinyl alcohol/polyacrylate salt series copolymer, an isobutylene/maleic anhydride series copolymer, an N-vinylacetamide series, an N-alkylacrylamide series copolymer, a starch/acrylate salt series copolymer, and the like. Specific and preferred examples thereof include a poly (meth) acrylic acid derivative, such as a polyacrylic acid alkali metal salt, a sodium (meth)acrylate-vinyl alcohol copolymer (a saponified product of a methyl (meth)acrylate-vinyl acetate copolymer), a saponified product of a poly(meth)acrylonitrile series copolymer, a hydroxyethyl methacrylate polymer, poly (meth) acrylamide and the like, an isobutylene-maleate salt series copolymer, a cellulose derivative, such as an alkali salt of carboxymethyl cellulose and the like, polyacrylamide, an alginic acid derivative, such as an alginic acid sodium salt, an alginic acid propylene glycol ester and the like, a starch derivative, such as starch sodium glycolate, starch sodium phosphate, a starch-acrylate salt graft copolymer and the like, a poly-N-vinylacetamide derivative, such as an N-vinylacetamide polymer and the like, an N-alkylacrylamide series copolymer, a polyvinyl alcohol derivative, such as polyvinyl alcohol, polyvinyl formal, polyvinyl acetal and the like, a PVA series-acrylate salt series copolymer, an isobutylene-maleic anhydride copolymer and a crosslinked product thereof, a crosslinked product of a vinyl alcohol-acrylate salt (containing a carboxyl group) copolymer, a neutralized product of a self-crosslinking polyacrylic acid, a crosslinked product of an acrylate salt series copolymer, a crosslinked product of a polyacrylic acid partially neutralized product, a crosslinked product of a polyacrylate salt, an acrylate ester-vinyl acetate copolymer and a saponified product of a crosslinked product thereof, a crosslinked product of an acrylate salt-acrylate ester copolymer, a crosslinked product of an acrylate salt-acrylamide copolymer, a crosslinked product of an acrylamide copolymer hydrolysate, a crosslinked product of a copolymer of 2-acrylamide-2-methylpropane sulfonate and acrylic acid, a hydrolysate of a polyacrylonitrile crosslinked product, a crosslinked product of an acrylonitrile copolymer hydrolysate, a crosslinked product of a starch-acrylic acid copolymer hydrolysate, a crosslinked product of a starch-acrylate salt copolymer, a crosslinked product of a starch-acrylonitrile copolymer and a crosslinked product of a hydrolysate thereof, a crosslinked product of a saponified product of a vinyl ester-ethylenic unsaturated carboxylic acid copolymer, a crosslinked product of a vinyl alcohol-maleic anhydride (cyclic anhydride) copolymer, an N--vinylacetamide polymer or copolymer, a crosslinked product of a carboxymethyl cellulose salt (a partial crosslinked product of a polysaccharide), a crosslinked product of a cationic monomer, a polyoxyethylene oxide crosslinked product, polyoxyethylene oxide crosslinked with acrylic acid, a crosslinked polyalkylene oxide, a crosslinked product of a copolymer of methoxypolyethylene glycol and acrylic acid, a polyacrylate salt crosslinked product, a crosslinked product of a starch-acrylic acid copolymer, a hydrolysate of a crosslinked product of a starch-acrylonitrile graft copolymer, a hydrolysate of an acrylate ester-vinyl acetate copolymer, a crosslinked product of an acrylate salt-acrylamide copolymer, and a hydrolysate of a polyacrylonitrile crosslinked product. In addition to the foregoing, examples thereof also include polyethylene oxide crosslinked with acrylic acid, a crosslinked product of sodium carboxycellulose, maleic anhydride-isobutylene, a copolymer obtained by copolymerizing acrylic acid with a comonomer, such as a maleate salt, an itaconate salt, a 2-acrylamide-2-methylsulfonate salt, a 2-acryloylethanesulfonate salt, a 2-hydroxyethyl acrylate and the like, and the like.

Examples of the water absorbing polymer that has biodegradability include a polyethylene oxide crosslinked product, a polyvinyl alcohol crosslinked product, a carboxymethyl cellulose crosslinked product, an alginic acid crosslinked product, a starch crosslinked product, a polyamino acid crosslinked product, a poly lactic acid crosslinked product and the like.

Examples thereof include one kind or a mixture of two or more kind selected from the foregoing. As for the shape of the water absorbing resin, those having various shapes, such as an irregular pulverized shape, a spherical shape, a substantially spherical shape, a squamous shape and the like, can be used.

Furthermore, it may be treated with a surfactant or used in combination with a surfactant, so as to improve the hydrophilicity.

Examples of the "salt" constituting various kinds of the high water absorbing resin exemplified as the water absorbing polymer include an alkali metal salt (such as a sodium salt, a potassium salt, a lithium salt and the like), an alkaline earth metal salt (such as a calcium salt, a magnesium salt, a barium salt and the like), an ammonium salt (such as a quaternary ammonium salt, a quaternary alkylammonium salt and the like), and the like.

Examples of a composite of resins having different water absorbing rates include a product obtained by adding second polymer particles 27B having a small water absorbing rate in an aqueous solution of a water soluble ethylenic unsaturated monomer, such as (meth) acrylic acid, 2-(meth)acrylamide-2-methylpropanesul fonic acid and the like, followed by subjecting to a polymerization reaction.

As the second polymer particles having a small water absorbing rate, a commercially available ordinary water absorbing resin may be used. Specific examples thereof include a starch series water absorbing resin, such as a hydrolysate of a starch-acrylonitrile graft copolymer, a neutralized product of a starch-acrylic acid graft copolymer and the like, a saponified product of a vinyl acetate-acrylate ester copolymer, a partially neutralized product of polyacrylic acid, a maleic anhydride-isobutylene copolymer, a polymer of a water soluble ethylenic unsaturated monomer, and the like.

The water retaining agent is not particularly limited as far as it can hold water, and examples thereof include wood powder, pulp powder, activated carbon, sawdust, cotton cloth having a large amount of cotton wool, short fibers of cotton, paper dusts, a vegetable material, a vegetable porous material having a capillary function and hydrophilicity, activated clay, a water-containing magnesium silicate clay mineral, such as zeolite and the like, perlite, vermiculite, a silica series porous substance, a coral fossil, a volcanic ash series substance (such as terraballoon, Shirasu balloon, Taisetsu balloon), and the like.

In particular, wood powder, terraballoon, Shirasu Balloon and Taisetsu balloon are preferred, and more preferably those containing 50% or more of particles having a particle diameter of 250 μm or less, further preferably 50% or more of particles having a particle diameter of 150 μm or less.

Those having been subjected to processing, such as baking and/or pulverization and the like, may be used in order to increase the water holding power of the water retaining agent and to enhance the shape retaining power.

Diatom earth, alumina, cellulose powder and the like may be added to the heat-generating composition of the invention depending on necessity. An anticaking agent may also be added.

Examples of the pH adjusting agent include a weak acid salt, a hydroxide and the like of an alkali metal, and a weak acid salt, a hydroxide and the like of an alkaline earth metal, and specific examples thereof include $Na_2CO_3$, $NaHCO_3$, $Na_3PO_4$, $Na_2HPO_4$, $Na_5P_3O_{10}$, NaOH, KOH, $CaCO_3$, $Ca(OH)_2$, $Mg(OH)_2$, $Ba(OH)_2$, $Ca_3(PO_4)_2$, $Ca(H_2PO_4)_2$ and the like.

The hydrogen generation suppressing agent may be any material that suppresses generation of hydrogen, and examples thereof include one kind or a material formed from two or more kinds selected from a metallic sulfide, such as calcium sulfide and the like, an oxidizing agent, an alkaline substance, sulfur, antimony, selenium, phosphorus and tellurium, and the foregoing pH adjusting agent. It is more effective by mixing it with the metallic powder as a heat-generating agent in advance since the addition amount can be reduced.

Examples of the oxidizing agent include a nitrate, a nitrite, an oxide, a peroxide, a halogenated oxyacid salt, a permanganic acid salt, a chromic acid salt and the like, and examples thereof include $NaNO_3$, $KNO_3$, $NaNO_2$, $KNO_2$, CuO, $MnO_2$ and the like.

Examples of the alkali substance include a silicate, a borate, a dibasic phosphate, tribasic phosphate, a sulfite, a thiosulfate, a carbonate, a hydrogen carbonate, $Na_2SiO_3$, $Na_4SiO_4$, $NaBO_4$, $Ba_2B_4O_7$, $KBO_2$, $Na_2HPO_4$, $Na_2SO_3$, $K_2SO_3$, $Na_2S_2O_3$, $Na_2CO_3$, $NaHCO_3$, $K_2S_2O_3$, $CaS_2O_3$, $Na_3PO_4$, $Na_5P_3O$ and the like.

In the case where the hydrogen generation suppressing agent is used in combination, examples thereof include a combination of an alkali weak acid salt and an alkali weak acid salt, such as $Na_2SO_3$-$Na_2SiO_3$, $Na_2SO_3$-$Na_2SiO_3$, $Na_2SO_3$-$Na_2B_4O_7$, $Na_2SO_3$-$CaOH_2$, $Na_2B_4O_7$-$Na_3PO_3$ and $Na_2CO_3$-$Na_2SO_2$, and a combination of an oxidizing agent and an alkali weak acid salt, such as $Na_3PO_4$-$Na_2SO_3$, S-$Na_2SO_3$, S-$Na_2S_2O_3$ and the like.

The using amount of the hydrogen generation suppressing agent in terms of a total amount of the respective hydrogen generation suppressing agents is preferably from 0.01 to 12.0% by weight, more preferably from 0.05 to 8% by weight, and further preferably from 0.5 to 2.0% by weight, based on the iron powder. When it is less than 0.01% by weight, the effect of suppressing generation of hydrogen is poor, and when it exceeds 12.0% by weight, it is not suitable since the heat-generation temperature is lowered while the effect of suppressing hydrogen generation is exerted.

The addition method is preferably addition in the form of an aqueous solution from the standpoint of workability and uniformity upon mixing, but even when it is added in a solid state separately from water, the effect of suppressing generation of hydrogen is substantially not differ from the case of the aqueous solution.

The far infrared ray radiating substance may be any substance that radiates a far infrared ray, and examples thereof include ceramics, alumina, zeolite, zirconium, silica and the like, one kind solely or a mixture of two or more kinds among which may be used.

The negative ion-generating substance may be any substance that directly or indirectly generates a negative ion as a result. Examples thereof include tourmaline, granite, Rochelle salt, glycin sulfate, potassium phosphate, a ferroelectric substance, such as calcium strontium propionate and the like, negatively ionized Si, $SiO_2$, devidite, brannerite, an excitation agent, such as feldspar and the like, a mineral ore containing a radioactive substance, such as radon and the like, and the like, and one kind solely or mixture of two or more kinds among these may be used. It is further effective that a material having a hydroxyl group is used in combination or retained by the base material or the like.

Examples of the pyroelectric substance include dravite, schorl, elbaite and tourmaline, such as rubellite, pink, baraiba, indecolite, water melon and the like. One kind solely or a mixture of two or more of dravite, schorl, elbaite and the like may be used.

Examples of the fertilizer component include a natural fertilizer, such as crushed bone, a mineral fertilizer and the like, a chemical fertilizer, such as urea, ammonium sulfate, ammonium chloride, superphosphate of lime, concentrated superphosphate of lime, potassium chloride, potassium sulfate, calcium chloride, calcium sulfate and the like, and one kind thereof solely, a mixed fertilizer obtained by mixing two or more kinds thereof or the like may be used, with those suitably containing the three elements, i.e., nitrogen, phosphoric acid and potassium, being preferred. Moreover, those having charcoal, ash contents and the like, which have such effects as a growth controlling function of saprophytic bacteria, a neutralizing and improving function of soil property and the like function, added thereto may be used.

Examples of the heat-generating assistant include metallic powder, a metallic salt, a metallic oxide and the like, and examples thereof include Cu, Mn, $CuCl_2$, $FeCl_2$, $FeCl_3$, $CuSO_4$, $FeSO_4$, CuO, manganese dioxide, cupric oxide, triiron tetroxide, a mixture thereof and the like.

The hydrophobic polymer compound may be any polymer compound that has a contact angle with water of 40° or more, more preferably 50° or more, and further preferably 60° or more, for improving water drainage of the composition. The shape thereof is not particularly limited, and examples thereof include a powder form, a granular form, a particulate form, a tablet form and the like, with a powder form, a granular form and a particulate form being preferred.

Examples thereof include a polyolefin, such as polyethylene, polypropylene and the like, a polyester, such as polyethylene terephthalate and the like, a polyamide, such as nylon and the like, and the like.

The organic silicon compoundmaybe anymaterial including a monomer, a low condensate, a polymer and the like, as far as it is a compound having a bond of Si-O-R and/or Si-N-R and/or Si-R', and examples thereof include an organic silane compound, such as a methyltrialkoxysilane, such as methyltriethoxysilane and the like, a tetraalkoxysilane, such as tetraethoxysilane and the like, and the like, a polyorganosiloxane (polysiloxane resin), such as a dimethylsilicone oil, a diphenylsilicone oil and the like, a cyclic siloxane, such as hexaorganocyclotrisiloxane and the like, a silicone resin composition containing the same, and the like.

The water-repelling agent can impart waterproofing property to the molded surface of the molded article of the invention by subjecting water-repelling treatment with a fluorine resin, an organic silicon compound or the like, and other various kinds of additives, such as an aromatic agent, an antifungal agent, an antibacterial agent, a coloring agent and the like may also be added in such a range that does not impair the properties of the molded article depending on necessity.

The surfactant encompasses anionic, cationic, nonionic and amphoteric surfactants. However, in the case where it is used, a nonionic surfactant is preferred.

Ethylene oxide, ethylene glycol, propylene oxide, propylene glycol and a polymer containing them are similarly useful as the additive.

Examples of the nonionic surfactant include polyoxyethylene alkyl ether, an ethylene oxide adduct of ricinus, an ethylene oxide adduct of alkylphenol, such as an ethylene oxide adduct of nonylphenol or octylphenol, and the like, a higher alcohol phosphate ester and the like.

Specific examples of other surfactants include a surfactant, such as sodium dodecylsulfate, sodium dodecylbenzenesulfonate and the like, and a silicone.

One kind solely or a mixture of two or more kinds among these may be used. A commercially available synthetic detergent containing these compounds may also be used.

Examples of the defoaming agent include an ordinary pH adjusting agent, such as sodium polyphosphate and the like, and other compounds that are used in this field of art are also used.

The aggregate may be any material that is useful for forming a porous body of the heat-generating composition, and examples thereof include silica-alumina powder, silica-magnesia powder, kaolin, colloidal silica, floatstone, silica gel, silica powder, mica powder, clay, talc, powder and pellets of a synthetic resin, a foamed synthetic resin, such as foamed polyester and polyurethane, and the like.

Examples of the binder include sodium silicate, sodium alginate, a polyvinyl acetate emulsion and the like.

Examples of the water soluble polymer include starch, gum arabic, methyl cellulose (MC), carboxymethyl cellulose (CMC), sodium carboxymethyl cellulose, polyvinyl alcohol (Poval) and the like.

Examples of the thickener include those that are generally used as a thickener, such as corn starch, bentonite and the like.

Examples of the excipient include those that are generally used as an excipient, such as sodium casein and the like.

Examples of the aggregating agent include those that are generally used as an excipient, such as corn syrup, mannite syrup and the like.

Examples of the soluble cohesive material include those that are generally used as an excipient, such as polyvinyl pyrrolidone and the like.

Examples of the foaming agent include any material that can generate a gas to attain foaming. Examples thereof include a decomposition-type foaming substance, which is a single substance that generates a gas through decomposition by heating, a reaction foaming-type agent, which generates a gas through reaction of two or more substances, and the like. The decomposition-type foaming agent is not particularly limited, and an inorganic decomposition foaming agent is preferably used. Representative examples thereof include sodium bicarbonate, ammonium carbonate, ammonium bicarbonate, ferrous carbonate and the like. It can also be used for such an object that it is foamed during the use as a heat-generating body to increase the air permeability and the moisture permeability. In addition, it can be appropriately selected as to whether or not heating is used for foaming.

Examples of the fibrous material include an inorganic fibrous material and/or an organic fibrous material. Examples thereof include rock wool, glass fibers, carbon fibers, asbestos fibers, boron fibers, alumina fibers, metallic fibers, natural fibers, such as pulp, paper, a nonwoven fabric, a woven fabric, cotton, linen and the like, regenerated fibers, such as rayon and the like, semisynthetic fibers, such as acetate and the like, synthetic fibers, and a pulverized product thereof.

The container bag in the invention is not particularly limited as far as it retains a mixture inside the bag, does not leak the raw materials upon using as a heat-generating body, has a strength that is sufficient for preventing from breakage, and has air permeability that is necessary for generating heat. The heat-generating body is not particularly limited in size and shape, and it may be in the form of a flat rectangular shape, a circular shape, a trapezoidal shape and the like corresponding to the place for using and the object of the use, and may also be in the form of a purse.

The position of the part of the container bag having air permeability may be any location as far as the heat-generating composition is pervaded with air. Examples thereof include the side that faces the human body, the side that does not face the human body, the side that is substantially in parallel to the human body, and the like.

The packing material is generally constituted with a base material, a covering material and the like, and it is not particularly limited as far as it is those that are generally used in heat-generating bodies of this kind, and includes a single layer material formed with a foamed or non-foamed film or sheet, and a laminated material formed by laminating plural layers in the thickness direction.

The packing material has at least one kind of property of air nonpermeability, air permeability, water nonabsorbing property, water absorbing property, nonelasticity, elasticity, non-heat sealing property, heat sealing property and the like.

In the container bag in the invention, there is no particular limitation in the case where the peripheral part of the heat-generating composition layer is sealed, and such a method is exemplified that a cohesive agent and/or an adhesive as a heat sealing material is provided on at least one of the base material, the covering material and a laying material, and the peripheral part of the heat-generating composition layer intervening at least between the base material and the covering material is sealed by cohesion, heat adhesion, heat fusing (heat sealing) and the like.

The heat-generating bag is not particularly limited in size and shape, and it may be in the form of a flat rectangular shape, a circular shape, a trapezoidal shape and the like corresponding to the place for using and the object of the use, and may also be in the form of a purse.

The thickness of the base material and the covering material largely varies depending on purposes and is not particularly limited.

Specifically, it is preferably from 5 to 5,000 μm, more preferably from 10 to 500 μm, and further preferably from 20 to 250 μm.

In the case where the film thickness of the packing material is less than 5 μm, it is not preferred since there is such a possibility that the necessary mechanical strength cannot be obtained, and a uniform film thickness cannot be obtained.

In the case where the film thickness of the packing material exceeds 5,000 μm, it is not preferred since the flexibility is lowered, so as to considerably deteriorate the fitness to the surface of the human body to be warmed and to deteriorate the followability to deformation and migration of the surface of the human body to be warmed, and also, the texture is deteriorated to cause stiffness, with the thickness of the total heat-generating body being too large.

Unevenness may be formed on at least one of the base material, the covering material and the heat-generating composition to prevent them from migration and deviation. That is, in the case where the surface of the base material and/or the covering material is smooth at least at the position of the base material and/or the covering material in contact with the heat-generating composition, it is possible that unevenness is physically formed on the surface thereof, or in alternative, a water absorbing material having water absorbing property is laminated on one surface or both surfaces of the base material and/or the covering material to form unevenness on the position of the base material and/or the covering material in contact with the heat-generating composition, whereby the bonding property to the heat-generating composition is increased through the adhesiveness associated with water absorption from the heat-generating composition and the unevenness, so as to prevent migration and deviation.

The packing material of the container bag may be a packing material having a multilayer structure, and while the structure is not particularly limited, examples thereof include a base material having two layers, layer A/layer B, or three layers, layer C/layer D/layer E, and a covering material having two layers, layer F/layer G, or three layers, layer H/layer I/layer J. The respective layers are laminated through a cohesive agent or an adhesive agent that is air permeable or air nonpermeable.

The layer A is such a layer that prevents exudation of the heat-generating composition and water content, and examples thereof include a waterproof film or sheet formed with a synthetic resin, such as a polyolefin and the like, e.g., linear low density polyethylene and the like.

The layer B, the layer E and the layer F are such a layer that has water absorbing property and air permeability, and examples thereof include a nonwoven fabric formed with a water absorbing material, such as a paper material, cotton, rayon and the like.

The layer C and the layer J are so-called reinforcing layers, and examples thereof include various kinds of nonwoven fabrics.

The layer D and the layer H are such layers that control air permeability and prevent exudation of the heat-generating composition, and examples thereof include an air permeable film or sheet formed with a synthetic resin, such as a polyolefin and the like.

Examples of the layer G include an air permeable or air nonpermeable film or sheet formed with a synthetic resin, such as polyolefin, polyester and the like.

The layer I is a so-called reinforcing layer, and examples thereof include a paper material.

Furthermore, in order to facilitate application of the heat-generating body for using and to prevent the heat-generating composition from migration and deviation, an antislipping layer or a non-transferable cohesive agent layer may be provided at least one surface thereof. Moreover, in the case where the antislipping layer or the cohesive agent layer is provided, it may be superimposed with releasing paper for protection until use.

The base material, the covering material and the cohesive agent layer each may be either transparent, opaque, colored, non-colored or the like. A layer constituting at least one layer among the layers constituting the respective materials and layers may be colored to such a color that is different from the other layers.

The heat-generating body thus obtained is sealed and stored in an air nonpermeable bag or the like to prevent from contacting with oxygen in air until use.

The air permeability of the container bag can be obtained by using an air permeable packing material on one surface or both surfaces of the bag. The air permeable packing material constituting the air permeable container bag is not particularly limited, and examples thereof include a material having air permeability formed by laminating a paper material on at least one kind of an air permeable film, a nonwoven fabric or the like, a material having air permeability formed by providing fine pores with needles in an air nonpermeable film, such as a polyethylene film and the like, a material having air permeability formed by providing fine pores with needles in an air nonpermeable packing material formed by laminating a nonwoven fabric on a polyethylene film, a nonwoven fabric controlled in air permeability by laminating and heat-fusing with fibers, a porous film, a material formed by laminating a porous film with a nonwoven fabric, and the like.

The air permeable packing material can be obtained by using in a part, one surface or both surface of the container bag.

The air permeability is not particularly limited as far as heat generation can be maintained, in the case where it is used as an ordinary chemical body warmer, the moisture permeability in terms of the Lyssy method is preferably from 50 to 10,000 $g/m^2 \cdot 24hr$, and more preferably from 100 to 5,000 $g/m^2 \cdot 24hr$.

In the case where the moisture permeability is less than 50, it is not preferred since the heat generation amount is small to fail to obtain a sufficient heating effect, and in the case where it exceeds 10,000 $g/m^2 \cdot 24hr$, there is a possibility that the heat generation temperature is increased to cause a problem on safety.

However, it is not limited depending on purposes that it may exceed 10, 000 $g/m^2 \cdot 24hr$, or a moisture permeability close to an open system may be used in some cases.

The air nonpermeable material constituting the air nonpermeable part of the container bag is not particularly limited as far as it is air nonpermeable, and examples thereof include a film, a sheet and a coated material of a synthetic resin, such as polyethylene, polypropylene, nylon, acryl, polyester, polyvinyl alcohol, polyurethane and the like, the foregoing hydrophobic polymers, and the like.

In the case where at least a part of free water, which is the excessive water content of the heat-generating composition, is absorbed by the base material and/or the covering material, the base material and/or the covering material are preferably formed with a water absorbing material, and the water absorbing material is not particularly limited as far as it has water absorbing property as a result, irrespective to as to whether or not the material itself has water absorbing property.

Examples thereof include a paper material, a paperboard, such as a corrugated fiberboard, a core of a corrugated fiberboard and the like, a foamed film or sheet having water absorption property (a foamed material, such as a water absorbing foamed polyurethane and the like), a nonwoven fabric or a woven fabric formed with fibers having water absorbing property, such as rayon and the like, cotton, pulp or the like, a nonwoven fabric or a woven fabric containing fibers having water nonabsorbing property, a material formed by containing, impregnating, kneading, transferring or carrying a water absorbing agent in a water absorbing porous film or sheet, a material formed by laminating a nonfoamed film or sheet, a foamed film or sheet, paper, such as waterproof paper and the like, a nonwoven fabric, a woven fabric or a porous film or sheet having water nonabsorbing property on a foamed film or sheet, paper, a nonwoven fabric, a woven fabric or a porous film or sheet having water absorbing property, a material obtained by laminating two or more kinds of them, and the like.

Furthermore, unevenness may be formed on the water absorbing material.

The water absorbing agent may be any material that has water absorbing property, and examples thereof include, in addition to the water retaining agent and a high water absorbing resin exemplified for the heat-generating composition, a water absorbing polymer that is generally employed.

In the case where high water absorbing fibers, such as vegetable fibers and the like, are used as a nonwoven fabric having water absorbing property, in the invention, the high water absorbing fibers preferably have a water absorbing capacity of 50 mL/g or more, and more preferably 100 mL/g or more.

As the water absorbing nonwoven fabric, that formed with high water absorbing fibers solely or that formed by blended spinning with other fibers. The kind of the fibers to be subjected to blended spinning with the high water absorbing fibers is not particularly limited, examples of which include synthetic fibers, such as polyethylene, polypropylene, nylon, acryl, polyester, polyvinyl alcohol, polyurethane and the like, natural fibers, such as cotton, pulp, viscous rayon and the like, and the like, and in the case where the both surfaces of the resulting heat-generating body are further covered with a film, a nonwoven fabric or the like, synthetic resin fibers, such as polyethylene, polypropylene, nylon, polyester and the like, are preferred.

Examples of the nonwoven fabric include a dry process nonwoven fabric, a wet process nonwoven fabric, a spunbond, a spunlace and the like. A nonwoven fabric formed with composite fibers having a core-shell structure may also be used.

Examples of the fibers constituting the woven fabric include natural fibers, regenerated fibers using a natural material, such as viscous fibers and the like, semisynthetic fibers, synthetic fibers, mixtures of two or more of them.

The water nonabsorbing material constituting the water nonabsorbing part of the container bag is not particularly limited as far as it has water nonabsorbing property, and examples thereof include a film, a sheet or a coated material formed with a synthetic resin, such as polyethylene, polypropylene, nylon, acryl, polyester, polyvinyl alcohol, polyurethane and the like, the foregoing hydrophobic polymers, and the like.

In the invention, in order that it is further preferably applied to a curved part, an expanding and contracting part, and further a bending part of the human body, and it further smoothly follows the expanding and contracting part and further a bending part, it is preferred that the base material and the covering material, i.e., the packing material of the heat-generating body for warming foot, are formed with an extensible film or sheet, particularly a stretch film or sheet.

The packing material having stretch property is not particularly limited, as far as it has stretch property. That is, it is sufficient that it has stretch property in total, and may be a single material or a composite material of a combination of stretch base materials, or a stretch base material and a non-stretch material.

Examples thereof include a single material of natural rubber, synthetic rubber, an elastomer, a stretch shape-memory polymer or the like, a mixture or a mixed yarn with a nonstretch material, a fabric, a film, a spandex thread, a thread, a string, a flat plate, a ribbon, a slit film, a foamed body and a nonwoven fabric constituted with a combination thereof, a composite stretch material formed by lamination or the like of these materials with a nonstretch material, and the like.

A heat sealing nonwoven fabric may be installed in the heat sealing container bag, and examples of the heat sealing nonwoven fabric include a composite nonwoven fabric of polyester and polyethylene.

Other examples of the heat sealing nonwoven fabric include a nonwoven fabric formed with fibers of a double structure, which contains a fiber core and a coated layer coated on the outer periphery of the core, in which the core is formed with polyester fibers or polypropylene fibers, and the coated layer is formed with polyethylene.

Furthermore, still other examples of the heat sealing nonwoven fabric include that using super fine spunbond obtained by dividing composite fibers, which contain polyethylene fibers and polyester superfine fibers surrounding thereon, in the axial direction of the fibers.

The film formed of a polyolefin series resin, the film formed of a polyurethane series resin and the film formed of a polyester series resin preferably has a thickness of from 5 to 500 μm, and more preferably from 10 to 350 μm, for exerting the prescribed mechanical strength and heat sealing property.

The paper material is not particularly limited, and examples thereof include paper and a paperboard. Examples thereof include one kind of or a laminated body of two or more kinds of thin paper, such as absorbing paper, tissue paper, crape paper and the like, packaging paper, such as craft paper and the like, multipurpose paper, such as paper for cards and the like, a corrugated fiberboard, a core of a corrugated fiberboard, such as a pulp core, a special core and the like, a liner of a corrugated fiberboard, such as craft, jute and the like, a paperboard, such as a coated paperboard and the like, building paper, such as base paper for a plaster board and the like, and the like.

The paper material may be subjected to a waterproof treatment, or may be adjusted in or imparted with air permeability, water absorbing property, air nonpermeability or water nonabsorbing property by providing through holes with a laser, a needle or the like depending on necessity.

Examples of the foamed sheet include a sheet formed with at least one kind selected from foamed polyurethane, foamed polystyrene, a foamed ABS resin, a foamed polyvinyl chloride, foamed polyethylene and foamed polypropylene.

The hot-melt adhesive is not particularly limited as far as it can be bonded by heating.

Examples of the hot-melt adhesive include an adhesive sheet formed with a hot-melt resin, such as an ethylene series hot-melt resin, such as an ethylene-acrylate ester copolymer resin, e.g., an ethylene-vinyl acetate copolymer resin, an ethylene-isobutyl acrylate copolymer resin and the like, and the like, a polyamide series hot-melt resin, a polyester series hot-melt resin, a butyral series hot-melt resin, a cellulose derivative series hot-melt resin, a polymethyl methacrylate series hot-melt resin, a polyvinyl ether series hot-melt resin, a polyurethane series hot-melt resin, a polycarbonate series hot-melt resin, vinyl acetate, a vinyl chloride-vinyl acetate copolymer, and the like.

Examples of the hot-melt resin also include those mixed with various kinds of antioxidants.

The hot-melt cohesive agent may be any material that can be subjected to melt-blowing and exhibits cohesiveness at ordinary temperature but can be melted by heating.

Examples thereof include a styrene series elastomer, such as SIS, SBS, SEBS and SIPS, an acrylic series elastomer containing an alkyl ester component, such as acrylic acid, methacrylic acid and the like, an olefin series elastomer, such as polyethylene, very low density polyethylene, polypropylene and an ethylene-vinyl acetate copolymer, a urethane series elastomer, and the like. These may be used solely or by mixing two or more kinds of them.

It is noted that the tackiness and the strength can be adjusted by adding an olefin series elastomer to a styrene series elastomer. Upon preparing the cohesive substance, appropriate additives, such as a tackifier, a softening agent, an antiaging agent and the like, may be mixed depending on necessity.

The cohesive agent for the cohesive agent layer is not particularly limited as far as it has such a fixing function that fixed through the cohesive force, and various forms thereof are used, such as a solvent series, an aqueous series, an emulsion type, a hot-melt type, a reactive type, a pressure-sensitive series and the like, examples of which include a vinyl acetate cohesive agent (a vinyl acetate resin series emulsion and an ethylene-vinyl acetate resin series hot-melt cohesive agent), a polyvinyl alcohol series cohesive agent, a polyvinyl acetal series cohesive agent, a vinyl chloride series cohesive agent, an acrylic series cohesive agent, a polyamide series cohesive agent, a polyethylene series cohesive agent, a cellulose series cohesive agent, a chloroprene (neoprene) series cohesive agent, a nitrile rubber series cohesive agent, a polysulfide series cohesive agent, a butyl rubber series cohesive agent, a silicone rubber series cohesive agent, a styrene series cohesive agent (such as a styrene series hot-melt cohesive agent), and the like.

In the case where a cohesive substance is obtained by kneading a substance radiating an infrared ray or the like with polyvinyl alcohol, the kind of the cohesive agent is not particularly limited, and it is kneaded with the foregoing cohesive agents.

As the gel layer, in addition to an aqueous gel layer constituted with a polyacrylic acid series aqueous gel, a cohesive layer formed by further mixing a water ab-sorbing polymer with the foregoing cohesive agent, i.e., a material using a cohesive layer formed with a hot-melt polymer substance, an alicyclic petroleum resin, a softening agent and a water absorbing polymer, is preferred from the standpoint of hygiene since a body fluid from the skin, such as sweat, secretions and the like, is absorbed and adsorbed by the water absorbing polymer to always maintain the surface of the outer skin clean.

As the gel layer in the invention, a material formed with from 5 to 40 parts by weight of a hot-melt polymer substance, from 5 to 55 parts by weight of an alicyclic petroleum resin, from 5 to 55 parts by weight of a softening agent, and from 0.5 to 10 parts by weight of a water absorbing polymer is useful, and particularly a material formed with from 10 to 30 parts by weight of a hot-melt polymer substance, from 10 to 50 parts by weight of an alicyclic petroleum resin, from 15 to 45 parts by weight of a softening agent, and from 1 to 8 parts by weight of a water absorbing polymer is further useful.

A surfactant may be added depending on necessity. The surfactant is not particularly limited as far as it facilitates dispersion of the water absorbing polymer in the cohesive layer of the cohesive agent, and examples thereof include an anionic surfactant, a cationic surfactant, a nonionic surfactant and an amphoteric surfactant.

The thickness of the cohesive agent layer or the gel layer is not particularly limited, and it is preferably from 5 to 1,000 μm, more preferably from 10 to 500 μm, and further preferably from 15 to 250 μm. In the case where the thickness of the cohesive agent layer is less than 5 μm, there are some cases where the necessary cohesive force cannot be obtained, whereas in the case where it exceeds 1, 000 μm, it is not preferred since not only it becomes bulky to impair application feeling, but also the economical efficiency is deteriorated.

The cohesive agent layer or the gel layer is generally provided over the entire surface, and a polymer having various shapes, such as a mesh shape, a stripe shape, a dot shape and the like, may be provided intermittently, so as to prevent occurrence of rubefaction, pain or the like upon using for a prolonged period.

The drug-containing layer may be any material that can contain a medical component, and a medical or sanitary agent maybe carried on at least one kind selected from the base material, the laying material, the covering material, the cohesive material layer, the gel layer and the antislipping layer.

The medical or sanitary agent may be any material that has an effect as a medical or sanitary agent, such as medical benefits. Examples thereof include a perfume material, such as peppermint, lavender oil and the like, medical plants, herb, an aromatic agent, a cosmetic lotion, a cosmetic latex, a wet compress, ginger extract, a Chinese medicine, a percutaneous absorption drug, an antifungal agent, an antibacterial agent, a disinfectant, an odor eliminating agent or a deodorizing agent, a magnetic material, a far infrared ray radiating substance, a negative ion-generating substance, a pyroelectric substance, such as tourmaline and the like, and the like.

The percutaneous absorption drug is not particularly limited, as far as it has percutaneous absorption property, and specific examples thereof include a skin stimulating drug, a paregoric and anti-inflammation drug, such as salicylic acid, indomethacin and the like, a central nerve acting drug (a sleeping and sedative drug, an antiepileptic drug and a neuropsychiatric drug), a diuretic drug, an antihypertension drug, a coronary vasodilating drug, an antitussive and expectoration drug, an antihistaminic drug, an antiarrhythmic drug, a cardiotonic drug, an adrenal cortex hormone drug, a topical anesthetic drug, and the like. These drugs may be used solely or by mixing two or more kinds of them depending on necessity.

The content of the drug is not particularly limited as far as it is in such a range that medical benefits are expected, and the content of the percutaneous absorption drug is preferably from 0.01 to 25 parts by weight, and more preferably from 0.5 to 15 parts by weight, per 100 parts by weight of the cohesive agent from the standpoint of pharmacologic effect and economy, and further from cohesive force.

The antibacterial agent, the disinfectant and the antifungal agent in the invention are not particularly limited as far as they exhibit a sterilization effect or a disinfectant effect or are effective for trichophytid, such as athlete's foot and the like, and specific examples thereof include a phenol derivative, salicilyc acid, boric acid, bleaching powder, an iodine drug, a heavy metal compound, inverted soap, an alifatic acid series substance, such as acetic acid, undecilic acid and the like, a salycilic acid series substance, a thianthol series substance, a tar series substance, a mercury series substance, such as phenylmercuric acetate and the like, sulfur, an antibiotic drug, polic, danba, asuretan, and the like.

The odor eliminating agent or the deodorizing agent in the invention may be those chemically decomposing components of odor through oxidation or reduction, and examples thereof include the following.

Examples thereof include such materials that are formed by containing a decomposing agent, such as a platinum group element, a compound thereof and the like, in a desiccating agent, such as aluminumoxide, silicon oxide, magnesiumoxide, titanium oxide, silica gel, zeolite, activated carbon and the like, and other carriers.

Other examples of the odor eliminating agent or the deodorizing agent in the invention include those countervailing odor by using an aromatic agent.

The heat-generating body thus obtained can be utilized for, in addition to warming in winter season, such affections as stiffness in shoulder, muscle ache, stiffness in muscle, lumbago, chill of limbs, neuralgia, rheumatism, bruise, sprain and the like, in which curative effects owing to heat is sufficiently expected, and it can further be utilized for heating and heat retention of machines, pets and the like and for a deoxidizer, an antifungal agent and the like.

According to these constitutions of the heat-generating body, shape retention is attained during the use of the heat-generating body to obtain high heat retention effects.

BRIEF DESCRIPTION OF THE DRAWINGS

Fig. 1 is a perspective view of an example of a heat-generating body of the invention.
Fig. 2 is a cross sectional view on line Z-Z.
Fig. 3 is a cross sectional view of another example of a heat-generating body of the invention.
Fig. 4 is a cross sectional view of still another example of a heat-generating body of the invention.
Fig. 5 is a cross sectional view of still another example of a heat-generating body of the invention.
Fig. 6 is a perspective view of still another example of a heat-generating body of the invention.
Fig. 7 is a schematic view of mold-through molding of a heat-generating body of the invention using a leveling plate.
Fig. 8 is an explanatory view of the vicinity of the leveling plate.
Fig. 9 is a schematic view of mold-through molding of a heat-generating body of the invention using a pressing and leveling plate.
Fig. 10 is a plane view of filter paper for measuring a water mobility value in the invention.
Fig. 11 is an explanatory view of measurement of a water mobility value in the invention.
Fig. 12 is an explanatory view of measurement of a water mobility value in the invention.
Fig. 13 is an explanatory view of measurement of a water mobility value in the invention.
Fig. 14 is an explanatory view of measurement of a water mobility value in the invention.
Fig. 15 is an explanatory view of a measuring method of a liquid permeability in the invention.
Fig. 16(a), (b) and (c) are explanatory views of measurement of a shape retaining degree in the invention.

The invention will be specifically described with reference to examples, but the invention is not limited thereto.

(Example 1)

100 parts by weight of iron powder (DKP, produced by Dowa Teppun Co., Ltd.), 8 parts by weight of activated carbon (SA-Super, produced by Norit Co., Ltd.), 4 parts by weight of sodium chloride, 0. 3 part by weight of a water absorbing polymer (KI-gel 201K, produced by Kraray Co., Ltd., particles having a particle diameter of 150 μm or more occupied 90% or more), 3.0 parts by weight of wood powder (particles having a particle diameter of 150 μm or less occupied 90% or more) and 0.15 part by weight of calcium hydroxide were mixed to produce a non-viscous heat-generating composition having a liquid permeation degree of 8, an incremental degree of viscosity of 200 cP and a water mobility value of 12 and having excessive water. The liquid permeation degree of the non-viscous heat-generating composition was 8.

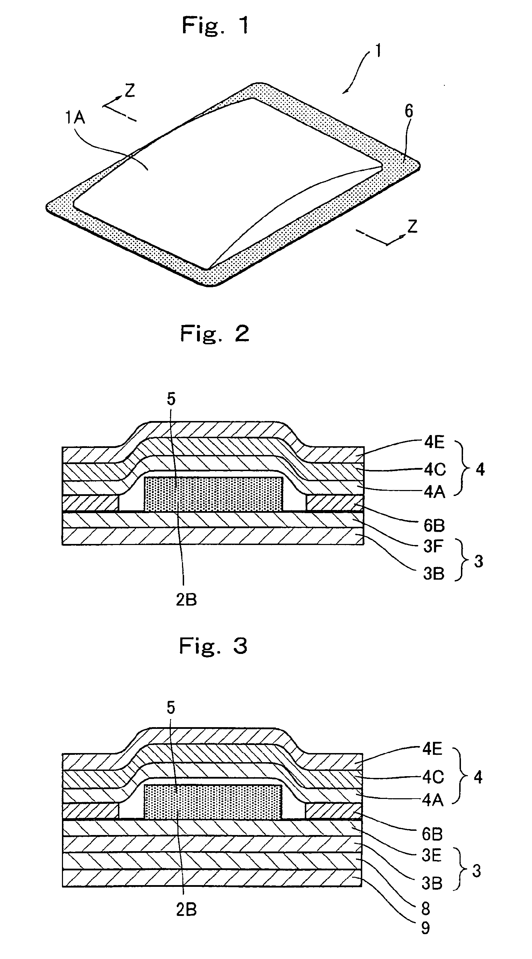

As shown in Figs. 1 and 2, an air nonpermeable packing material formed by laminating a polyethylene film 3B on line paper 3F was designated as a base material 3. A molded article of a heat-generating composition 2B obtained by molding the heat-generating composition by mold-through was laminated on the base material 3, and a hot-melt adhesive layer 6b was provided by a melt blowing method on an outer periphery of the molded article of the heat-generating composition 2B. An air permeable packing material formed by laminating a nylon nonwoven fabric 4E, a porous film 4C and craft paper 4A in this order was used as a covering material 4, which was laminated thereon in such a manner that the surface on the side of the craft paper 4A was in contact with the molded article of the heat-generating composition 2B, and then fixed by pressing on the adhesive agent layer 6B. Thereafter, it was cut into a rectangular shape having a length of 135 mm, a width of 100 mm and a seal width of 8 mm to produce a flat heat-generating body 1 shown in Fig. 1. The air permeability of the covering material 4 was 400 g/

m$^2$·24hr in terms of moisture permeability.

The heat-generating body was constituted in such a manner that a part of water content in the non-viscous heat-generating composition 2 having excessive water was absorbed by the base material 3 and/or the covering material 4 to remove the barrier layer, whereby the heat-generating composition 2 was formed into a porous body to facilitate contact with air. In the figure, 1A denotes a heat-generating part, and 6 denotes a sealing part.

The heat-generating body was charged and sealed in an air nonpermeable outer bag, and was allowed to stand at room temperature for 24 hours. After 24 hours, the heat-generating body was taken out from the outer bag and was subjected to a heat generation test, and it reached 36°C within 1 minute and exhibited a long heat-generating duration at 36°C or more of 8 hours.

(Comparative Example 1)

A non-viscous heat-generating composition having an incremental degree of viscosity of 500 cP was obtained in the same manner as in Example 1 except that in the formulation in Example 1, the water absorbing polymer was removed and replaced with 15 g of wood powder. The liquid permeation rate for an 11% saline solution was 36. The heat-generating composition had a water mobility value of 20. Thereafter, a heat-generating body having a length of 135 mm, a width of 100 mm and a seal width of 8 mm was produced in the same manner as in Example 1. The heat-generating body was placed and sealed in an air nonpermeable outer bag, and was allowed to stand at room temperature for 24 hours. After 24 hours, the heat-generating body was taken out from the outer bag and was subjected to a heat generation test, and it reached 36°C within 1 minute but exhibited a short heat-generating duration at 36°C or more of 4 hours.

(Comparative Example 2)

A viscous heat-generating composition having an incremental degree of viscosity exceeding 100,000 cP was obtained in the same manner as in Example 1 except that 1.5 parts by weight of CMC was added to the formulation in Example 1. The liquid permeation rate for an 11% saline solution was 4. The heat-generating composition had a water mobility value of 4. Thereafter, a heat-generating body having a length of 135 mm, a width of 100 mm and a seal width of 8 mm was produced in the same manner as in Example 1. The heat-generating body was placed and sealed in an air nonpermeable outer bag, and was allowed to stand at room temperature for 24 hours. After 24 hours, the heat-generating body was taken out from the outer bag and was subjected to a heat generation test, but excessive water was not completely discharged, and it required 15 minutes for reaching 36°C and exhibited a short heat-generating duration at 36°C or more of 3 hours.

(Comparative Example 3)

A heat-generating body (thickness of a molded article of the heat-generating composition: 1.7 mm) produced in the same manner as in Example 1 was subjected to a pressing roll to produce a heat-generating body having a thickness of a molded article of the heat-generating composition of 1.0 mm. The heat-generating body was placed and sealed in an air nonpermeable outer bag in the same manner as in Example 1, and was allowed to stand at room temperature for 24 hours. After 24 hours, the heat-generating body was taken out from the outer bag and was subjected to a heat generation test, but excessive water was not completely discharged, and it required 15 minutes for reaching 36°C and exhibited a short heat-generating duration at 36°C or more of 3 hours. The molded article of the heat-generating composition (thickness: 1.7 mm) in Example 1 produced by mold-through molding had a liquid permeation degree of 8, and a material formed by subjecting the same to a pressing roll to make a thickness of 1. 0 mm had a liquid permeation degree of 2.

(Example 2)

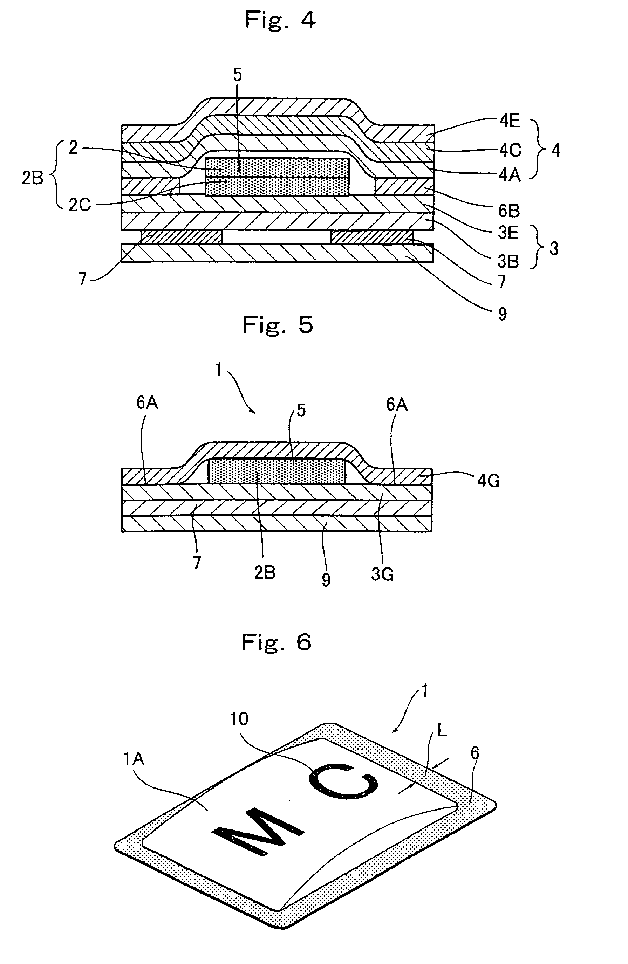

An assembly obtained by replacing the liner paper 3F of the base material 3 in Example 1 with craft paper 3E and laminating an antislipping layer 8 and a releasing film 9 below the craft paper is shown in Fig. 3. Fig. 4 shows a cross sectional view of an assembly formed by providing an acrylic adhesive agent layer 7 between the craft paper 3E and the releasing film 9.

(Example 3)

A heat-generating body was produced in the same manner as in Example 1 except that 3 parts by weight of terraballoon was added instead of the wood powder in Example 1. Furthermore, the heat-generating body was sealed in an air nonpermeable outer bag and was allowed to stand at room temperature for 24 hours.

After 24 hours, the heat-generating body was taken out from the outer bag, and the measurement for shape retaining degree revealed that it was 100. By using another heat-generating body produced in the same time, the heat-generating body was similarly taken out from the outer bag and was subjected to a heat generation test, and it reached 36°C within 1 minute and exhibited a long heat-generating duration at 36°C or more of 8 hours.

(Example 4)

A mixture of a high water absorbing resin containing 0.1 part by weight of a water absorbing polymer A (polyacrylate salt series, particle diameter: 150 to 300 μm) and 0.2 part by weight of a water absorbing polymer B (polyacrylate salt series, particle diameter: 64 μm or less), 100 parts by weight of iron powder, 5 parts by weight of activated carbon, 3 parts by weight of wood powder and 0.15 part by weight of calcium hydroxide were sufficiently mixed in a mixer, to which 55 parts by weight of a 11% sodium chloride aqueous solution was then added, followed by further mixing, so as to obtain a non-viscous heat-generating composition having excessive water of the invention. The heat-generating composition had a water mobility value of 10. Thereafter, it was molded by mold-through molding to a thickness of 1.5 mm on core paper of a corrugated fiberboard of a base material formed by laminating the core paper for a corrugated fiberboard with a polyethylene film, and then an air permeable covering material was laid thereon, followed by sealing the peripheral part of the heat-generating composition, so as to produce a heat-generating body having a seal width of 10 mm, a width of 90 mm and a length of 250 mm. The heat-generating body was charged and sealed in an air nonpermeable outer bag and was allowed to stand at room temperature for 24 hours. After lapsing 24 hours, the heat-generating body was taken out from the outer bag and was subjected to a heat generation test, and it reached 36°C within 1 minute and exhibited a long heat-generating duration at 36°C or more of 8 hours.

(Example 5)

0.3 part by weight of a water absorbing polymer (polyacrylate salt series, particle diameter: 64 μm or less), 8.0 parts by weight of activated carbon, 4.0 parts by weight sodium chloride, 0.15 part by weight of calcium hydroxide, 0.3 part by weight of sodium sulfite and 50 parts by weight of water were added to 100 parts by weight of iron powder as a heat-generating substance, and they were kneaded and prepared.

That is, the foregoing formulation having iron powder, activated carbon, water absorbing polymer, sodium chloride, pH adjusting agent and hydrogen generation suppressing agent was put in a mixer (capacity: 100 L) in this order and agitated for 5 minutes, and a sodium chloride aqueous solution was added under agitation and kneaded for 10 minutes, followed by discharging. The resulting non-viscous heat-generating composition having excessive water 5 had an incremental degree of viscosity of 500 cP, a liquid permeation degree of 6 and a water mobility value of 12.

As the base material, a water absorbing heat sealing base material 3G, which was an air nonpermeable laminated film, was used that was formed by laminating a nonwoven fabric layer (basis weight: 50 $g/m^2$) having hydrophobicity and heat sealing property formed with polyester fibers and polyethylene fibers, a composite laminated nonwoven fabric (basis weight: 50 $g/m^2$) of a water absorbing cotton nonwoven fabric and a high density polyethylene film having a thickness of 40 μm sandwiched by a low density polyethylene resin having a thickness of 40 μm, followed by providing a cohesive agent layer with a releasing film on the side of the high density polyethylene film.

As the covering material, a water absorbing heat sealing covering material 4G, which was a laminated film, was used that was formed in such a manner that a polyester nonwoven fabric having a basis weight of 30 $g/m^2$ and a polyethylene porous film having a thickness of 40 μm were adhered and laminated in this order from the exposed surface with a hot-melt adhesive of a styrene-isoprene-styrene block copolymer over the entire surface with a pattern having adhered parts of 1 mm and nonadhered parts of 1 mm at an angle of 45°, and then a hydrophilic and water absorbing cotton nonwoven fabric having a basis weight of 50 $g/m^2$ and a hydrophobic and heat sealing composite spunbonded nonwoven fabric of polyester fibers and polyethylene fibers having a basis weight of 50 $g/m^2$ were sequentially adhered in the similar adhesion method (provided that the adhesion pattern crossed the previous pattern at right angles). The moisture permeability of the covering material 8 was 400 $g/m^2 \cdot 24hr$ in terms of the Lyssy method of the moisture permeability of the covering material.

A non-viscous heat-generating composition having excessive water 5 similar to Example 1 was laminated to a rectangular shape having a thickness of 1.5 mm by mold-through molding by using a through mold having a thickness of 1.5 mm at a prescribed position on the heat sealing composite nonwoven fabric of the base material 3G, and the covering material 4 was overlaid thereon in such a manner that the water absorbing and heat sealing composite nonwoven fabric of the covering material 4G was in contact with the molded article of the heat-generating composition 2B. The base material and the covering material were heat-sealed at a periphery of the molded article of the heat-generating composition 2B, and cut into a prescribed size to produce a heat-generating body 1 shown in Fig. 5. 6A denotes the heat sealing part. A cohesive agent layer 7 and a releasing film 9 were provided on the back surface of the base material 3G. Subsequently, it was sealed in an outer bag having airtightness not shown in the figure.

After lapsing 24 hours from sealing in the outer bag, it was used by breaking the outer bag, and thus the heat-generating temperature was increased to about 36°C within about 1 minute, and generated heat at from 36 to 41°C for about 7 hours.

The heat-generating body was constituted in such a manner that a part of the water content of the non-viscous heat-generating composition having excessive water 5 was absorbed by the cotton nonwoven fabric in the base material and the cotton nonwoven fabric in the covering material 4G to remove the barrier layer, whereby the heat-generating composition 5 was formed in to a porous body to facilitate contact with air.

The heat-generating composition 5 is in the form of sherbet having a small surface area to restrict the contact area with air, and further, the free water functions as a barrier layer suppressing contact of the iron powder with air, whereby the oxidation amount per unit time is especially restricted. As a result, a covering material in the form of a film or a sheet is laminated thereon, and thus an oxidation reaction is substantially blocked until the heat-generating sheet is obtained.

(Example 6)

A heat-generating body having a adhesive agent layer was produced in the same manner as in Example 4 except that the base material of Example 5 was changed to a base material having a hot-melt styrene series adhesive agent layer, and the covering material was changed to a covering material having figures. The heat generation test was carried out, and the similar results as in Example 5 were obtained.

(Example 7)

100 parts by weight of iron powder, 0.3 part by weight of a water absorbing polymer, 3 parts by weight of wood powder, 8 parts by weight of activated carbon, 6 parts by weight of sodium chloride, 0.25 part by weight of sodium polyphosphate and 45 parts by weight of water were placed in a V blender in the order of activated carbon, water absorbing polymer, wood powder, sodium chloride, sodium polyphosphate and iron powder, and after agitating and mixing for 15 minutes, water was gradually added under agitation. After further agitating and mixing for 15 minutes, discharge was carried out to obtain a non-viscous heat-generating composition having excessive water. The liquid permeation degree was 8.

As the base material 7, an air nonpermeable laminated film was used that was formed by laminating a hydrophobic and heat sealing nonwoven fabric layer (basis weight: 50 g/m$^2$) formed with polyester fibers and polyethylene fibers and a polyester nonwoven fabric (basis weight: 30 g/m$^2$) sandwiched by a polyethylene resin having a thickness of 40 $\mu$m.

As the covering material 8, a laminated film was used that was formed in such a manner that a polyester nonwoven fabric having a basis weight of 30 g/m$^2$ and a polyethylene porous film having a thickness of 40 $\mu$m were adhered and laminated in this order from the exposed surface with a hot-melt adhesive of a styrene-isoprene-styrene block copolymer over the entire surface with a pattern having adhered parts of 1 mm and nonadhered parts of 1 mm at an angle of 45°, and then a hydrophobic and heat sealed composite spunbonded nonwoven fabric of polyester fibers and polyethylene fibers having a basis weight of 50 g/m$^2$ was sequentially adhered in the similar adhesion method (provided that the adhesion pattern crossed the previous pattern at right angles). The moisture permeability of the covering material 8 was 410 g/m$^2$·24hr in terms of the Lyssy method of the moisture permeability of the covering material. Figures of MC were provided on the covering material.

A heat-generating composition 2 in a sherbet form was laminated to a rectangular shape having a thickness of 1.5 mm by mold-through molding by using a through mold having a thickness of 1.5 mm punched into a rectangular shape, and after removing the punched plate, at a prescribed position on a hydrophobic and heat sealing nonwoven fabric layer (basis weight: 50 g/m$^2$) formed with polyester fibers and polyethylene fibers was overlaid thereon. After dehydrating through pressing rolls, excess nonwoven fabric was cut and removed to obtain a rectangular molded article of the heat-generating composition in the heat-generating condition.

Subsequently, it was molded in the same manner as in Example 1, the sealing parts of the base material 3 and the covering material 4 were heat sealed at the outer periphery of the molded article of the heat-generating composition 2B, followed by cutting to make a circumferential sealing width L of 5 mm, so as to produce a heat-generating body (Fig. 6) having a cohesive agent layer containing a adhesive agent layer (thickness: 25 $\mu$m) not shown in the figure formed with an acrylic cohesive agent over the entire surface of one surface of the flat packing material having air permeability. In the figure, 1A denotes a heat-generating part, 6 denotes a sealing part. A releasing film for protecting the surface of the cohesive agent layer was provided on the cohesive agent layer. The heat-generating body had a shape maintenance degree of 100. After producing the heat-generating body, it was charged in an air nonpermeable outer bag.

After lapsing 24 hours from charging, it was used by breaking the outer bag, and thus the heat generation temperature was increased to about 36°C within about 30 seconds and was maintained at from 36 to 41°C over about 6 hours.

(Example 8)

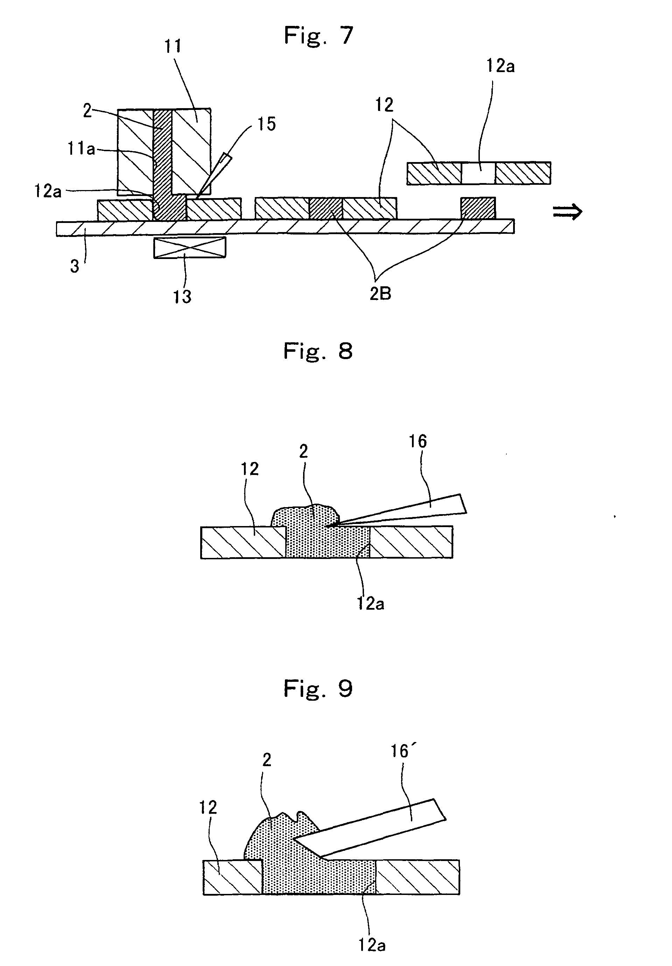

Figs. 7 and 8 show an example of a mold-through molding method using a leveling plate 16. That is, a base material 3 having a width of 130 mm in the form of a roll film is aligned on a mold 12 for molding of a thickness of 1 mm having a desired shape punched on the center of the mold, and the assembly is horizontally conveyed at a prescribed speed between a dice 11 on the upper surface and a magnet 13 on the lower surface. A heat-generating composition 2 in the form of sherbet according to the invention is fed from the upper surface of the mold 12 to a mold hole 12a through a hole 11a of the dice 11. The heat-generating composition 2 is leveled to the same level as the mold 12 with a leveling plate 16 arranged in front of the conveying direction and simultaneously charged in the mold hole 12a to form a shape having a thickness of 1.5 mm on the base material 3. Thereafter, the mold 12 is removed to obtain a molded article laminated on the base material 3.

While not shown in the figures, thereafter, a cohesive polymer of a styrene-isoprene-styrene block copolymer (SIS) series is provided by a melt-blowing method in the form of mesh on the surface of the molded article, and a covering material is overlaid thereon and sealed by heat sealing at the periphery of the molded article, followed by cutting into a prescribed shape, so as to obtain a heat-generating body having a desired shape. Furthermore, the cut heat-generating body of the invention is subsequently fed to a packaging step and sealed in an outer bag having airtightness. The similar molding can be carried out even when the leveling plate 16 is replaced with a pressing and leveling plate 16'. Fig. 8 shows the leveling plate 16, and Fig. 9 shows the pressing and leveling plate 16'. If the pressing and leveling function is maintained, the tip end part of the pressing and leveling plate may be subjected to any deformation, such as trimming to form roundness, i.e., a curved surface, and the like.

INDUSTRIAL APPLICABILITY

(1) The non-viscous heat-generating composition having excessive water of the invention contains, as essential components, a water absorbing polymer, a carbon component, a heat-generating promoter, water and a heat-generating substance and has a liquid permeation degree of 5 or more, and thus the excessive water can be easily drained. Therefore, heat generation can be immediately started by breaking an airtight bag for storage upon using, whereby a desired heat generation temperature can be rapidly obtained, and heat generation can be maintained for a long period of time.

(2) The molded article of the non-viscous heat-generating composition having excessive water of the invention has a shape maintenance degree of 70 or more, and in the case where the inner pressure of the container bag becomes larger than the outer pressure, it is deformed, and any of a punched film, a porous film material capable of adjusting pressure and a punched film that is difficult to adjust pressure can be used. Therefore, the selection range of the air permeable material is broadened, whereby the cost can be reduced, and a body to be warmed can be uniformly warmed at an appropriate temperature for a long period of time.

(3) Owing to the heat-generating composition having excessive water, it is considerably high in flowability, moldability and shape retaining property in comparison to the conventional powder heat-generating composition. Therefore, it can be continuously and uniformly laminated on an accurate position of a base material that is conveyed at a high speed, for example, of 50 m or more per minute, by mold-through molding, printing or the like, and various kinds of shapes of a rectangular shape, a circular shape or the like can be produced with from an ultrathin shape to a thick form.

(4) Owing to the heat-generating composition having excessive water, radiation of powder of a heat-generating composition to the environment as in the conventional technique is prevented, and thus such factory administration can be carried out that completely satisfies the intending GMP standard for medical devices and production of medical drugs in the future.

As described in the foregoing, the heat-generating body having excellent characteristics that have not been found in the conventional heat-generating body can be obtained.

**Claims**

1.  A non-viscous heat-generating composition having excessive water , **characterized in that** the heat-generating composition generating heat in contact with air comprises, as essential components, a water absorbing polymer, a carbon component, a heat-generating accelerator, water and a heat-generating substance, has excessive water, and has an incremental degree of viscosity of 1,000 cP or less and a liquid permeability of 5 or more.

2.  A non-viscous heat-generating composition having excessive water as described in claim 1, **characterized in that** the non-viscous heat-generating composition having excessive water contains a water retaining agent.

3.  A non-viscous heat-generating composition having excessive water as described in claim 1, **characterized in that** the water retaining agent is at least one kind selected from wood powder, terraballoon and Shirasu balloon.

4.  A non-viscous heat-generating composition having excessive water as described in claim 1, **characterized in that** the non-viscous heat-generating composition having excessive water contains at least one kind selected from a surfactant, a defoaming agent, a pH adjusting agent, a hydrophobic polymer compound, a pyroelectric substance, a far infrared ray radiating substance, a negative ion-generating substance, a hydrogen generation suppressing substance, an organic silicon compound, a water soluble polymer, a thickener, a binder, an excipient, an aggregating agent, a soluble adhesive material, an aggregate, a fibrous material, a medical or sanitary agent, a fertilizer component and a heat-generating assistant.

5. A non-viscous heat-generating composition having excessive water as described in claim 1, **characterized in that** water insoluble solid components other than a high water absorbing resin in the non-viscous heat-generating composition having excessive water has an average particle diameter of 150 μm or less.

6. A non-viscous heat-generating composition having excessive water as described in claim 1, **characterized in that** the non-viscous heat-generating composition having excessive water has a shape retaining degree of 70 or more.

7. A heat-generating body **characterized by** placing a non-viscous heat-generating composition having excessive water as described in claim 1 in a container bag having air permeability in at least a part thereof.

8. A heat-generating body as described in claim 7, **characterized in that** a molded article of the heat-generating composition in the heat-generating body has a shape retaining degree of 70 or more.

9. A heat-generating body as described in claim 7, **characterized in that** at least a part of the container bag has water absorbing property.

10. A heat-generating body as described in claim 7, **characterized in that** at least one kind selected from iron powder, a carbon component, a fibrous material, a binder, a thickener, an excipient, an aggregating agent, a soluble cohesive material, a far infrared ray radiating substance, a negative ion-generating substance, a pyroelectric substance, an organic silicon compound, a water absorbing agent, a water absorbing polymer, a water-separation preventing stabilizer and a medical or sanitary agent is laminated, diffused or coated on one surface or both surfaces of a molded article of the heat-generating composition.

11. A heat-generating body as described in claim 7, **characterized in that** at least one kind selected from a far infrared ray radiating substance, a negative ion-generating material, a pyroelectric substance and a medical or sanitary agent is laminated with, coated on or contained in at least one kind or at least a part of a base material and a covering material constituting the container bag.

12. A heat-generating body as described in claim 7, **characterized in that** unevenness is formed on the whole surface or a part surface of a molded article of the heat-generating composition.

13. A heat-generating body as described in claim 7, **characterized in that** unevenness is formed on at least the whole surface or a part surface of a molded article of the heat-generating composition and a material laminated on the heat-generating composition.

14. A heat-generating body as described in claim 7, **characterized in that** a adhesive agent layer or a gel layer is laminated on at least a part of an exposed surface of one of the base material and the covering material.

15. A heat-generating body as described in claim 14, **characterized in that** the adhesive agent layer or the gel layer is a wet compress layer containing a wet compress agent, or a drug-containing layer laminated with, coated with, containing or carrying at least one kind of a far infrared ray radiating substance, a negative ion-generating substance, a pyroelectric substance and a medical or sanitary agent.

## Fig. 1

## Fig. 2

## Fig. 3

## Fig. 4

## Fig. 5

## Fig. 6

Fig. 7

Fig. 8

Fig. 9

## Fig. 10

## Fig. 11

## Fig. 12

## Fig. 13

## Fig. 14

## Fig. 15

## Fig. 16(a)

## Fig. 16(b)

### BEFORE TEST

## Fig. 16(c)

### AFTER TEST

COMPOSITION

# EP 1 516 900 A1

## INTERNATIONAL SEARCH REPORT

International application No.

PCT/JP03/04363

### A. CLASSIFICATION OF SUBJECT MATTER
Int.Cl$^7$ C09K5/16, A61F7/08

According to International Patent Classification (IPC) or to both national classification and IPC

### B. FIELDS SEARCHED

Minimum documentation searched (classification system followed by classification symbols)
Int.Cl$^7$ C09K5/16, A61F7/08

Documentation searched other than minimum documentation to the extent that such documents are included in the fields searched

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X<br>Y | EP 1121912 A2 (JAPAN PIONICS CO., LTD.),<br>08 August, 2001 (08.08.01),<br>Claim 1; column 4, lines 39 to 41;<br>Par. Nos. [0044] to [0045]; examples 1 to 5<br>& JP 2001-212167 A<br>Claim 1; column 5, lines 12 to 13; Par. Nos. [0028],<br>[0036] to [0037]; examples 1 to 5 | 1-10,14-15<br>11-13 |
| X | JP 2001-89757 A (Kao Corp.),<br>03 April, 2001 (03.04.01),<br>Claims 1 to 2; examples 1 to 3<br>(Family: none) | 1-9 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>09 June, 2003 (09.06.03) | Date of mailing of the international search report<br>24 June, 2003 (24.06.03) |
|---|---|
| Name and mailing address of the ISA/<br>Japanese Patent Office | Authorized officer |
| Facsimile No. | Telephone No. |

Form PCT/ISA/210 (second sheet) (July 1998)

28

**INTERNATIONAL SEARCH REPORT**

International application No.

PCT/JP03/04363

C (Continuation). DOCUMENTS CONSIDERED TO BE RELEVANT

| Category* | Citation of document, with indication, where appropriate, of the relevant passages | Relevant to claim No. |
|---|---|---|
| Y | GB 2303208 A (Akio USUI),<br>12 February, 1997 (12.02.97),<br>Page 45, line 14 to page 46, line 3;<br>page 59, lines 21 to 25<br>& JP 9-75388 A<br>Par. Nos. [0114], [0163] | 11-13 |
| A | US 6099556 A (Akio USUI),<br>08 August, 2000 (08.08.00),<br>Claims 1 to 17<br>& JP 9-48968 A<br>Claims 1 to 19 | 1-15 |

Form PCT/ISA/210 (continuation of second sheet) (July 1998)